# EUROPÄISCHE PATENTSCHRIFT

(11) **EP 3 706 393 B1**
(45) Veröffentlichungstag und Bekanntmachung des Hinweises auf die Patenterteilung: **24.04.2024**
(21) Anmeldenummer: 19160516.1
(22) Anmeldetag: 04.03.2019
(51) Int. Cl.: H04L 67/025, H04L 67/04, H04L 65/80, H04L 67/08, H04L 67/565, H04L 67/5651, H04L 67/61, H04L 67/63

(54) **VERFAHREN ZUR ÜBERTRAGUNG EINER BENUTZEROBERFLÄCHE, MEDIZINGERÄT UND ANORDNUNG**
METHOD FOR TRANSMITTING A USER INTERFACE, MEDICAL DEVICE, AND SYSTEM
PROCÉDÉ DE TRANSMISSION D'UNE INTERFACE UTILISATEUR, APPAREIL MÉDICAL ET SYSTÈME

(43) Veröffentlichungstag der Anmeldung: 09.09.2020
(73) Patentinhaber: Siemens Healthineers AG, 91301 Forchheim (DE)
(72) Erfinder: Schweizer, Hans, 94447 Plattling (DE)
(74) Vertreter: Siemens Healthineers Patent Attorneys

(56) Entgegenhaltungen:
- DE-A1-102014 219 760
- US-A1- 2018 302 594
- WEIDONG SHI ET AL: "Scalable Support for 3D Graphics Applications in Cloud", CLOUD COMPUTING (CLOUD), 2010 IEEE 3RD INTERNATIONAL CONFERENCE ON, IEEE, PISCATAWAY, NJ, USA, 5 July 2010 (2010-07-05), pages 346-353, XP031739415, ISBN: 978-1-4244-8207-8

## Beschreibung

Die Erfindung betrifft ein Verfahren zur Übertragung einer Benutzeroberfläche sowie ein Medizingerät und einen Server.

In einem Krankenhaus kommen typischerweise eine Vielzahl unterschiedlicher Geräte, d.h. Medizingeräte zur Anwendung, welche regelmäßig auch von unterschiedlichen Anbietern hergestellt sind. Beispiele für solche Medizingeräte sind bildgebende Geräte, speziell C-Bögen oder Ultraschallgeräte, aber auch Navigationsgeräte, Geräte zur Überwachung klinischer Parameter eines Patienten oder Geräte für Eingriffe an einem Patienten. Bei der Nutzung mehrerer Geräte in einer einzelnen Anwendung, z.B. einer konkreten Operation, ist ein Anwender der Geräte, z.B. ein Chirurg oder sonstiges OP-Personal, auf Interaktionen mit diesen mehreren Geräten angewiesen. Daraus ergibt sich, dass für ein möglichst optimales klinisches Ergebnis ein möglichst reibungsloses Zusammenspiel dieser Geräte von entscheidender Bedeutung ist.

Problematisch ist zunächst, dass die vielen Geräte um den begrenzten Platz in der Nähe des Patienten, speziell um einen OP-Tisch herum konkurrieren, da jedes der Geräte eine oder mehrere Mensch-Maschine-Schnittstellen aufweist, welche für den Anwender in Reichweite sein müssen. Solche Schnittstellen sind z.B. Monitore zur Anzeige sowie Tastaturen oder Mäuse zur Eingabe oder Touchscreens, d.h. spezielle Monitore, sowohl zur Anzeige als auch zur Eingabe. Regelmäßig wird zur Interaktion eine Benutzeroberfläche auf einem Monitor dargestellt, welche zugleich zur Anzeige von Informationen sowie zur Eingabe von Steuerbefehlen mittels eines Eingabegeräts dient. Eine Zusammenlegung der Schnittstellen mehrerer Geräte zwecks Platzeinsparung und Reduzierung der Anzeige- und Eingabemodalitäten im Bereich des OP-Tisches ist dabei nicht ohne Weiteres möglich, da in den einzelnen Geräten üblicherweise aus einer Risikoklassifizierung heraus Maßnahmen implementiert sind, welche an die jeweilige Schnittstelle gebunden sind. Zusätzlich besteht auch das Problem, dass die diversen Geräte regelmäßig geräteeigene Kabelverbindungen aufweisen, was speziell im sterilen, klinischen Umfeld und genauer in einer OP-Umgebung zu besonderen hygienischen Herausforderungen führt.

Besonders beim Betrieb von Medizingeräten können sich Verzögerungen bei der Anzeige von Informationen sowie bei der Eingabe von Steuerbefehlen fatal auswirken. Beispielsweise ist bei einer Wirbelsäulenoperation die Navigation von chirurgischem Werkzeug gestützt durch eine Anzeige, deren genaue Darstellung entsprechend zeitkritisch ist. So erfolgt das Einsetzen einer Pedikelschraube in einen Wirbelkörper beispielsweise durch Beobachtung über einen Monitor, welcher entsprechend verlässlich den aktuellen Zustand anzeigen muss, da ansonsten der Patient schwer und nachhaltig verletzt werden könnte.

Die Druckschrift DE 10 2014 219 760 A1 offenbart einen Server verbunden mit einem tragbaren Informationssystem, einem mobilen Client und einem Bedienpanel. Bediendaten werden auf dem mobilen Client oder dem Bedienpanel angezeigt.

Aus der Publikation WEIDONG SHI ET AL: "Scalable Support for 3D Graphics Applications in Cloud", CLOUD COMPUTING (CLOUD), 2010 IEEE 3RD INTERNATIONAL CONFERENCE ON, IEEE, PISCATAWAY, NJ, USA, 5. Juli 2010 (2010-07-05), Seiten 346-353, XP031739415, ISBN: 978-1-4244-8207-8 ist eine Software-Architektur bekannt, die in skalierbarer Weise Anwendungen der 3D Bildbearbeitung in der Cloud ermöglicht. Die Software-Architektur ermöglicht es, Medien-Daten zu Clients in der Cloud zu streamen. Dabei werden Latenzzeiten der Übertragung von Video-Frames zwischen Client und Server gemessen.

Die Druckschrift US 2018/302594 A1 offenbart ein System zur Verarbeitung von Videoeingaben mit einem Medien-Auswahlschalter an einem ersten Ort, einer oder mehreren Videosignalquellen an einem zweiten Ort und einem Steuerclient an einem dritten Ort. Der Steuerclient ist über ein öffentliches Netzwerk mit dem Medien-Auswahlschalter verbunden und bietet einem Benutzer eine Schnittstelle zur Auswahl eines oder mehrerer Videosignale aus den Quellen. Ein Latenzüberwachungselement ermöglicht die Überwachung der Latenz verschiedener Netzwerkpfade des öffentlichen Netzwerks, um die Latenz der Quellsignale zu minimieren.

Vor diesem Hintergrund ist es eine Aufgabe, die Benutzeroberfläche eines Geräts zwecks Anzeige von Informationen dieses Geräts und zwecks Steuerung dieses Geräts an ein anderes Gerät zu übertragen. Die Übertragung der Benutzeroberfläche soll mit einer möglichst einfachen und kostengünstigen Technologie erfolgen und gleichzeitig aber eine möglichst risikoarme Steuerung des Geräts gewährleisten, speziell vor dem Hintergrund einer möglicherweise verzögerten Übertragung. Insbesondere sollen auch die Benutzeroberflächen von mehreren Geräten gebündelt werden, um dadurch den Verkabelungsaufwand speziell in einem OP möglichst gering zu halten.

Die Aufgabe wird erfindungsgemäß gelöst durch ein Verfahren mit den Merkmalen gemäß Anspruch 1, durch ein Medizingerät mit den Merkmalen gemäß Anspruch 8 sowie durch eine Anordnung mit den Merkmalen gemäß Anspruch 9. Vorteilhafte Ausgestaltungen, Weiterbildungen und Varianten sind Gegenstand der Unteransprüche. Dabei gelten die Ausführungen im Zusammenhang mit dem Verfahren sinngemäß auch für das Medizingerät sowie für die Anordnung und umgekehrt.

Das Verfahren dient zur Übertragung einer Benutzeroberfläche von wenigstens einem Server über einen Proxy auf wenigstens einen Client. Der Proxy und sämtliche Clients sind insbesondere Bestandteile desselben Geräts und dabei über wenigstens ein internes Netzwerk miteinander verbunden. Das Gerät mit Proxy und Client wird auch als Master-Gerät bezeichnet. Der Proxy bildet eine Schnittstelle des Geräts nach außen und ist dann über wenigstens ein externes Netzwerk mit einem oder mehreren anderen Geräten verbunden, welche jeweils einen Server darstellen. Die Ausführungen zu Ausgestaltungen mit einem Server oder mit einem Client gelten sinngemäß auch für Ausgestaltungen mit mehreren Servern oder mehreren Clients. Das interne Netzwerk ist bevorzugt ein TCP/IP-Netzwerk. Der Proxy ist zwischen Server und Client geschaltet, sodass sämtliche Daten, welche von einem Server zu einem Client oder umgekehrt übertragen werden durch den Proxy laufen. Dadurch ist die Benutzeroberfläche eines jeden Servers auf jedem der Clients zugleich darstellbar und umgekehrt sind analog auch mehrere Server von einem einzelnen Client aus steuerbar.

Das Master-Gerät ist ausgebildet zur Durchführung des Verfahrens und weist hierzu insbesondere ein Steuergerät auf, auch als Controller bezeichnet, welches das Verfahren ausführt. Das Steuergerät ist z.B. ein Computer, welcher auch den Proxy enthält. In einer bevorzugten Ausgestaltung ist das Master-Gerät ein Medizingerät, vorzugsweise ein bildgebendes Medizingerät, besonders bevorzugt ein C-Bogen. Ein bildgebendes Medizingerät weist prinzipbedingt wenigstens einen Monitor auf, welcher dann vorliegend als Client dient, um alternativ oder in Kombination mit einer Benutzeroberfläche des Master-Geräts selbst auch eine oder mehrere Benutzeroberflächen eines oder mehrerer Server anzuzeigen. Besonders zweckmäßig ist ein Touchscreen als Client.

In einer geeigneten Ausgestaltung ist das Master-Gerät mehrteilig ausgebildet und weist eine Haupteinheit und eine Nebeneinheit auf. Bei einem C-Bogen weist die Haupteinheit dann einen C-Arm und eine Strahlenquelle auf, sowie ein Hauptsteuergerät, z.B. das oben genannte Steuergerät, und einen Monitor, speziell zur Steuerung des Master-Geräts. Die Nebeneinheit ist dann ein Beiwagen, welcher separat von der Haupteinheit positionierbar ist und einen oder mehrere weitere Monitore aufweist, speziell insbesondere zur Darstellung von Bildern, welche mit dem Master-Gerät aufgenommen werden. Die Monitore von Haupt- und Nebengerät werden dann jeweils als Clients verwendet. Der Server ist vorzugsweise ebenfalls ein Medizingerät, insbesondere jedoch von einem anderen Hersteller, d.h. von einem Fremdanbieter. Beispielsweise ist der Server ein Navigationsgerät, zur Abbildung der verschiedenen Positionen diverser Objekte in einem gemeinsamen Koordinatensystem, um z.B. einen Chirurgen bei der Planung einer Operation zu unterstützen. Solche Objekte sind beispielsweise ein Patient, ein oder mehrere Medizingeräte, insbesondere ein bildgebendes Medizingerät, ein Instrument zur Behandlung oder für einen Eingriff oder ein Implantat oder dergleichen.

Gemäß dem Verfahren wird eine Benutzeroberfläche des Servers an den Client derart übertragen, dass die Benutzeroberfläche auf dem Client angezeigt wird und der Server vom Client aus steuerbar ist. Dadurch ist es für einen Anwender möglich, mittels des Clients den Server zu steuern und umgekehrt Informationen des Servers an den Client zu übermitteln und über diesen auszugeben. Die Benutzeroberfläche wird auch als GUI (graphical user interface) bezeichnet und umfasst typischerweise Anzeigefelder zur Darstellung von Informationen sowie Bedienfelder zur Eingabe von Steuerbefehlen. Durch die Übermittlung der Benutzeroberfläche ist diese auf dem Client nutzbar, um den Server fernzusteuern. Die konkrete Ausgestaltung der Benutzeroberfläche ist vom Gerät abhängig. Über die Benutzeroberfläche nimmt der Client eine Eingabe, z.B. einen Steuerbefehl, eines Anwenders auf. Die Eingabe wird dann über den Proxy an den Server weitergeleitet und von diesem verarbeitet. Umgekehrt wird eine optische, akustische oder haptische Ausgabe des Servers über den Proxy an den Client geleitet und dort an den Anwender ausgegeben. Allgemein ist also vom Client aus eine Interaktion mit dem Server möglich, wobei unter "Interaktion" zusammenfassend eine Ausgabe von Informationen an einen Anwender einerseits und eine Eingabe des Anwenders zur Steuerung des Geräts andererseits verstanden werden.

Gemäß dem Verfahren wird zur Anzeige der Benutzeroberfläche auf dem Client ein Datenstrom von mehreren aufeinanderfolgenden Bildern vom Server an den Client mittels einer paketbasierten Übertragung übermittelt. Die Bilder sind Bilder zur Darstellung der Benutzeroberfläche und werden vom Server erzeugt. Der Server weist insbesondere selbst ein Anzeigegerät auf, zur Anzeige der Benutzeroberfläche zwecks direkter Steuerung des Servers. Vorliegend werden die Bilder aber alternativ oder zusätzlich an den Client übermittelt. Dies erfolgt vorliegend in Form von Paketen. Grundsätzlich ist es denkbar, dass einzelne Bilder in mehreren Paketen übertragen werden, welche nach der Übertragung wieder zusammengesetzt werden, um das Bild zu erhalten, z.B. bei Verwendung von TCP/IP, welches Pakete auf eine bestimmte Größe begrenzt. Vorliegend wird jedoch vereinfachend und ohne Beschränkung der Allgemeinheit davon ausgegangen, dass ein einzelnes Bild zu genau einem Paket gehört, dass also ein einzelnes Paket ein einzelnes Bild enthält. Der Datenstrom besteht demnach aus mehreren aufeinanderfolgenden Paketen, welche mit einer Frequenz ausgesendet werden, welche einer Bildwiederholrate des Servers entspricht. Ein Paket enthält zusätzlich zu einem Bild insbesondere noch andere Daten, z.B. eine Bildnummer sowie andere Parameter, welche sich aus der weiteren Beschreibung ergeben. Ein Paket wird alternativ auch als Telegramm bezeichnet.

Die paketbasierte Übertragung zwischen Server und Client ist insbesondere bidirektional. Der Server versendet ein Paket mit einem Bild und ggf. zusätzlichen anderen Daten über den Proxy an den Client. Nach Empfang des Pakets sendet der Client ein Paket als eine Rückantwort über den Proxy an den Server. Die Rückantwort enthält insbesondere eine Bildnummer, sodass der Server die Rückantwort einem zuvor versendeten Paket zuordnen kann. Server, Proxy und Client sind jeweils ausgebildet die Pakete in beiden Richtungen zu empfangen, auszuwerten und ggf. Daten zu ändern, zu löschen oder hinzuzufügen.

Gemäß dem Verfahren wird zur Überwachung der Übertragung eine Latenz der Übertragung wiederkehrend für einzelne Bilder bestimmt, zweckmäßigerweise für jedes Bild. Durch diese wiederkehrende Überwachung des Empfangs der einzelnen Bilder, d.h. insbesondere der einzelnen Pakete, ist eine Überwachung der Latenz der Übertragung auf Bildspeicherebene (framebuffer level) realisiert. Da die Latenz prinzipbedingt bestimmt, welche Zeit zur Übermittlung der Bilder benötigt wird, lässt sich durch eine Beobachtung der Ankunft der Bilddaten am Client die Latenz bestimmen, d.h. insbesondere messen oder quantifizieren. Vorliegend wird daher die Übermittlung eines Bildes oder allgemeiner eines Pakets für eine Bestimmung der Latenz verwendet, und zwar wiederkehrend, sodass fortlaufend die Latenz bestimmt wird. Geeigneterweise wird dann abhängig von der Latenz eine geeignete Reaktion des Servers, des Proxy oder des Clients veranlasst.

Ein besonderer Vorteil der Erfindung besteht insbesondere darin, dass die Latenz mit einer Frequenz gemessen wird, welche in der Größenordnung der Bildwiederholrate des Servers und des Clients liegt. Bevorzugterweise wird bei jedem Empfang eines Bildes die entsprechende Latenz bestimmt, sodass die Latenz also bildweise gemessen wird, wodurch sich eine entsprechende Messrate ergibt. Wie weiter unten im Detail ausgeführt wird, wird die Bildwiederholrate in einer vorteilhaften Weiterbildung nicht vollständig vom Proxy an den Client weitergegeben, sondern reduziert, sodass sich bei einer Bestimmung der Latenz durch den Client die Messrate unter Umständen entsprechend reduziert. Nichtsdestoweniger erfolgt die Bestimmung der Latenz bei beispielhaften Bildwiederholraten von 30 bis 60 Bilder/s damit üblicherweise im Abstand von wenigen zehn Millisekunden. Insgesamt ist eine Verlangsamung der Übertragung somit besonders schnell erkennbar und entsprechend eine Reaktion hierauf besonders zeitnah möglich. Dies ist speziell im klinischen Umfeld vorteilhaft, da sich hier Verzögerungen und daraus resultierende Fehlinformationen besonders drastisch auswirken.

Ein weiterer Vorteil ist insbesondere, dass die Bestimmung der Latenz vorliegend protokollseitig definiert ist, also durch ein Übertragungsprotokoll, sodass keine speziellen Anforderungen an die Hardware vorliegen und das Verfahren überwiegend oder sogar vollständig mit besonders kostengünstiger und einfacher Standard-Technologie durchführbar ist und insbesondere auch durchgeführt wird. Unter einem Übertragungsprotokoll werden vorliegend insbesondere bestimmte Vorschriften, d.h. Verfahrensschritte zur Handhabung der Pakete durch den Server, den Proxy und den Client verstanden sowie insbesondere auch eine bestimmte Zusammensetzung der Pakete. Zumindest im externen Netzwerk zwischen Server und Proxy erfolgt die Übertragung der Benutzeroberfläche drahtgebunden oder drahtlos über ein kostengünstiges, paketbasiertes Netzwerk, vorzugsweise Standard-Ethernet, WLAN oder dergleichen, und gerade nicht über ein spezielles Echtzeit-Ethernet. Das interne Netzwerk basiert beispielsweise auf TCP- oder UDP-Technologie oder ähnlichen Protokollen, welche auf Standard-Ethernet-Hardware laufen. Das Verfahren und das hierbei angewendete Übertragungsprotokoll zeichnen sich dadurch aus, dass eine Überwachung der Latenz ausschließlich oder zumindest überwiegend softwareseitig realisiert ist und daher keine spezielle Hardware benötigt wird, sodass kostengünstige Standardkomponenten verwendet werden, z.B. Cat-5-Kabel. Die beteiligte Hardware, also Server, Proxy und Client müssen lediglich zur Anwendung des Übertragungsprotokolls ausgebildet sein, d.h. entsprechende Programme ausführen, durch welche die Ausführung des Verfahrens veranlasst wird. Dadurch, dass das Übertragungsprotokoll auf der Bildspeicherebene arbeitet, werden vorteilhaft jegliche fensterbasierten Umgebungen unterstützt. Insbesondere dient das Übertragungsprotokoll auch zur Übermittlung von Steuerbefehlen an den Server über einen entsprechenden Rückkanal.

Die Erfindung basiert zunächst insbesondere auf der Beobachtung, dass eine einfache Videoverteillösung grundsätzlich geeignet ist, um eine Benutzeroberfläche eines Geräts auf ein anderes Gerät zu übertragen und auch, um mehrere Benutzeroberflächen auf einem Gerät zu bündeln. Hierzu wird das jeweilige Monitorsignal hardwareseitig abgegriffen. In die unter Umständen proprietäre Software des entsprechenden Geräts braucht dabei nicht eingegriffen zu werden, sondern es wird sozusagen lediglich dessen Ausgabe kopiert. Diese Lösung ist besonders bei Geräten von Fremdanbietern vorteilhaft. Nachteilig ist bei der Videoverteillösung allerdings die zusätzlich notwendige Hardware, nämlich eine zusätzliche Verkabelung, sowie das prinzipbedingte Fehlen eines Rückkanals zur Steuerung des Geräts. Für einen solchen Rückkanal sind im Falle einer einfachen Videoverteillösung zusätzliche Kabel und auch zusätzliche Eingabegeräte notwendig. Die Übertragung einer Benutzeroberfläche eines Geräts an ein anderes Gerät bei vollem Funktionserhalt ist daher im Rahmen einer einfachen Videoverteillösung nicht möglich.

Der Server ist ausgebildet zur Durchführung des Verfahrens, insbesondere in Kombination mit einem entsprechenden Master-Gerät mit Proxy und Client. Zur Durchführung des Verfahrens weist der Server insbesondere ein Steuergerät auf, auch als Controller bezeichnet, z.B. einen Computer.

Vorzugsweise werden die Bilder des Servers softwareseitig abgegriffen, z.B. mittels eines auf dem Server hierfür gespeicherten Serverprogramms, z.B. einer DLL (dynamic link library). Dabei wird mittels dieses Serverprogramms die gesamte serverseitige Bildversendung durchgeführt, insbesondere bestehend aus einem Einlesen (grabbing) der Bilder aus einem Bildspeicher des Servers, optional einer Kodierung der Bilder sowie die paketbasierte Versendung der Bilder zum Proxy und letztendlich zum Client. Dabei wird z.B. ein vom Hersteller vordefinierter Anzeigebereich des Bildspeichers ausgelesen und versendet. Vorzugsweise wird mittels desselben Serverprogramms auch umgekehrt eine Steuerung des Servers realisiert, indem Steuerbefehle an den Server empfangen und an diesen ausgegeben werden.

In einer vorteilhaften Variante werden die Bilder des Servers hardwareseitig abgegriffen, dabei jedoch ohne Eingriff in die Hardware des Servers. Dies wird dadurch realisiert, dass ein separater, d.h. eigenständiger Computer, auf welchem das oben beschriebene Serverprogramm ausgeführt wird, über einen Bildausgang an den Server angeschlossen ist und auf diese Weise dann die Bilder aus dem Bildspeicher einliest, ggf. kodiert und schließlich über den Proxy an den Client versendet. Der separate Computer wird somit als nicht-invasiver Grabber genutzt.

Auch der Rückkanal wird zweckmäßigerweise durch das beschriebene Serverprogramm auf dem Server oder auf einem separaten Computer bereitgestellt. Entsprechend erfolgt hierüber die Versendung von Steuerbefehlen vom Client an den Server. Im Falle des hardwareseitigen Abgreifens mittels separatem Computer ist dieser geeigneterweise über einen entsprechenden Anschluss zur Eingabe der Steuerbefehle mit dem Server verbunden, z.B. über einen USB-Anschluss. Der separate Computer verhält sich dann zum Server insbesondere wie ein Eingabegerät, z.B. wie eine Tastatur oder Maus.

Insgesamt ist dann sowohl bei rein softwareseitiger Umsetzung als auch bei Verwendung eines separaten Computers vorteilhaft eine bidirektionale Verbindung von Server und Client realisiert, ohne in die Software oder die Hardware des Servers selbst eingreifen zu müssen. Die Aufgabe wird somit auch durch das beschriebene Serverprogramm gelöst, welches beim Ausführen auf einem Server oder auf einem separaten Computer, welcher an einen Server angeschlossen ist, diesen veranlasst, das Übertragungsprotokoll umzusetzen, d.h. die den Server betreffenden Schritte des Verfahrens auszuführen, insbesondere in Verbindung mit einem entsprechenden Proxy und Client. Ein solches Serverprogramm wird daher auch als erfinderisch angesehen.

Ebenso wird die Aufgabe gelöst durch ein Clientprogramm, welches beim Ausführen auf einem Master-Gerät, insbesondere einem Client eines Master-Geräts, dieses veranlasst, das Übertragungsprotokoll umzusetzen, d.h. die den Client und vorzugsweise auch die den Proxy betreffenden Schritte des Verfahrens auszuführen, insbesondere in Verbindung mit einem entsprechenden Server. Ein solches Clientprogramm wird daher auch als erfinderisch angesehen.

Das Serverprogramm und das Clientprogramm sind jeweils Computerprogramme. Entsprechend wird die Aufgabe auch gelöst durch einen computerlesbaren Datenträger, auf welchem das Serverprogramm oder das Clientprogramm gespeichert ist.

Weiter geht die Erfindung von der Beobachtung aus, dass sich zur Weiterleitung der Eingabe von einem Gerät zu einem anderen Gerät eine Fernsteuerung eignet, beispielsweise basierend auf Remote Desktop oder einem Terminal Server. Die jeweilige Anzeige und Eingabe werden über eine paketbasierte Verbindung übertragen, wodurch es möglich ist auch eine Eingabefunktionalität der Benutzeroberfläche zu erhalten. Hierbei ergeben sich jedoch je nach Netzwerk oftmals Bandbreitenbeschränkungen. Auch ist durch das Netzwerk nicht unbedingt die z.B. hinsichtlich Verzögerung einer Anzeige geforderte Sicherheit gewährleistet, d.h. es erfolgt keine Überwachung der Latenz der Übertragung zwischen den Geräten, was aber zur Gewährleistung der Aktualität von Anzeige und Eingabe besonders im klinischen Umfeld wichtig ist. Spezielle Echtzeitnetzwerk-Technologien, z.B. Profinet (Markenname), stellen zwar eine bestimmte Antwortzeit sicher, sind jedoch kostenintensiv und erfordern insbesondere auch hardwareseitige Modifikationen, z.B. die Verwendung von Switches.

Insgesamt besteht somit ein Kernaspekt der Erfindung insbesondere darin, dass eine besonders sichere Übertragung einer Benutzeroberfläche in einem möglichst einfachen Standard-Netzwerk durch eine protokollseitig definierte Latenzüberwachung auf Bildspeicherebene realisiert ist.

Die Latenz, d.h. die Verzögerung bei der Übertragung von Bilddaten vom Server zum Client, wird vorliegend bestimmt, um daraufhin den Server, den Client oder den Proxy zu einer geeigneten Reaktion hierauf zu veranlassen. Dabei wird eine Reaktion zweckmäßigerweise bei Überschreiten eines vorgegebenen Schwellwerts für die Latenz ausgelöst. Die Latenz wird vom Client und vom Proxy bestimmt. Die hierzu notwendigen Informationen entnimmt der Client, der Proxy oder der Server dem Datenstrom, insbesondere den einzelnen Paketen.

Vorliegend wird die Latenz für den jeweiligen Datenstrom vom Client bestimmt und von diesem auch genutzt, um dessen Verhalten gegenüber dem Anwender geeignet anzupassen. Dem liegt die Überlegung zugrunde, dass der Client die Schnittstelle zum Anwender darstellt und daher sinnvollerweise genutzt wird, um den Anwender über Übertragungsprobleme zu informieren und Fehleingaben direkt zu verhindern.

Gemäß der Erfindung wird die Latenz als absolute Latenz, kurz AFL (absolute frame latency) bestimmt, nämlich als Differenz zwischen einem Beginn einer serverseitigen Verarbeitung eines jeweiligen Bildes und dem Ende einer clientseitigen Verarbeitung für das jeweilige Bild.

In einer bevorzugten Ausgestaltung sendet der Server beim Übertragen eines jeweiligen Bildes einen Zeitstempel mit, welcher eine Startzeit der serverseitigen Verarbeitung des Bildes angibt, nämlich den Beginn eines Einlesens des Bildes aus einem Bildspeicher des Servers. Die clientseitige Verarbeitung des Bildes ist in einen Empfangsvorgang und einen Anzeigevorgang für das Bild unterteilt, wobei der Anzeigevorgang insbesondere erst dann beginnt, wenn der Empfangsvorgang beendet ist. Die absolute Latenz wird dann als Summe aus zwei Zeitabschnitten bestimmt, nämlich einem ersten Zeitabschnitt, welcher von der Startzeit bis zum Ende des Empfangsvorgangs reicht, wobei dieses Ende insbesondere durch eine Endzeit markiert ist, welche entsprechend festgestellt wird. Ein zweiter Zeitabschnitt gibt eine Dauer des Anzeigevorgangs an. Die Endzeit wird vorzugsweise nach einem Empfang des jeweiligen Bildes durch den Client selbst festgestellt. Insgesamt umfasst die absolute Latenz somit die serverseitige und die clientseitige Bildverarbeitung sowie die Übertragung über den Proxy und die beteiligten Netzwerke.

Zur Bestimmung der absoluten Latenz wird vom Client eine serverseitige Information benötigt, nämlich die Startzeit, welche vom Server entsprechend mit dem jeweiligen Bild übermittelt wird. Die absolute Latenz oder geeignete Parameter für deren Bestimmung werden vom Client zweckmäßigerweise zusammen mit der entsprechenden Bildnummer der Rückantwort über den Proxy an den Server beigefügt, sodass dieser dann selbst die absolute Latenz bestimmen kann.

Die absolute Latenz ergibt sich vorzugsweise aus den folgenden, nacheinander ausgeführten Schritten und insbesondere lediglich aus der Summe der Dauern dieser Schritte: erstens, ein serverseitiger Einlesevorgang für ein Bild, nämlich ein Einlesen (grabben) des Bildes aus dem Bildspeicher des Servers; zweitens, eine Komprimierung des Bildes, welche allerdings optional ist und in einer Variante ausgelassen wird; drittens, eine paketbasierte Übertragung des Bildes vom Server über den Proxy zum Client; viertens, ein Empfang des Bildes durch den Client, insbesondere mit einer zusätzlichen Dekodierung des Bildes; fünftens, eine Anzeige des Bildes auf dem Client durch entsprechende Ansteuerung eines Monitors und geeignete Umschaltung von Pixeln. Der erste und zweite Schritt sind dabei Bestandteile der serverseitigen Verarbeitung des Bildes. Der dritte Schritt bezeichnet die Übertragung zwischen Server und Client über den Proxy und die diversen Netzwerke. Der vierte und fünfte Schritt sind Bestandteile der clientseitigen Verarbeitung des Bildes, wobei der vierte Schritt dem Empfangsvorgang des Clients entspricht und der fünfte Schritte dem Anzeigevorgang. Jeder dieser Schritte weist eine gewisse Dauer auf und die Summe dieser Dauern ergibt die absolute Latenz.

Die Dauer vom Beginn des ersten Schritts bis zum Ende des vierten Schritts, also vom Beginn des Einlesevorgangs bis zum Ende des Empfangsvorgangs wird vorzugsweise anhand der Startzeit und der Endzeit errechnet und somit insbesondere vollständig softwareseitig. Die Bestimmung der Dauer des fünften Schritts, d.h. des Anzeigevorgangs, ist demgegenüber nicht ohne Weiteres mit Standardmitteln erfassbar, da diese Dauer regelmäßig insbesondere vom Grafiktreiber oder Displaycontroller des Clients abhängig ist, wobei z.B. die vertikale Synchronisation oder die Doppelpufferung von Bildern von Bedeutung sind.

In einer geeigneten Ausgestaltung ist die Dauer des Anzeigevorgangs als ein vorbestimmter Offset in einem Speicher hinterlegt, insbesondere in einem Speicher des Clients. Der Offset wird z.B. im Vorfeld durch entsprechende Versuche und Messungen ermittelt und dann im Speicher hinterlegt und im laufenden Betrieb dann bei Bedarf nur noch abgefragt. Die Bestimmung der absoluten Latenz ist somit vorteilhaft vollständig softwareseitig realisiert.

In einer ebenfalls geeigneten Variante wird die Dauer des Anzeigevorgangs vom Client für ein jeweiliges Bild gemessen. Hierzu wird der Client vorzugsweise mit einem speziellen Anzeigetreiber betrieben, welcher laufend bei einem jeweiligen Bild dynamisch die Dauer des Anzeigevorgangs misst, insbesondere in Kombination mit einer geeignet angepassten Firmware für einen Displaycontroller des Clients. Zweckmäßigerweise wird hierbei eine Aktivität einer Hintergrundbeleuchtung eines Monitors, d.h. Displays des Clients überwacht, um sicherzustellen, dass das jeweilige Bild auch tatsächlich für den Anwender sichtbar geworden ist. Die Bestimmung der Latenz wird damit nochmals deutlich verbessert und eignet sich dann für besonders sicherheitskritische Anwendungen. Im Gegensatz zur zuvor beschriebenen, rein softwareseitigen Bestimmung, weist in dieser Ausgestaltung die Bestimmung der absoluten Latenz dann eine hardwareseitige Anpassung auf, nämlich die beschriebene Modifikation des Clients, welche allerdings vorteilhaft den Server, also das Gerät eines potentiellen Fremdanbieters, weiterhin unberührt lässt. Abgesehen hiervon ist aber in dieser Ausgestaltung die Bestimmung der absoluten Latenz im Übrigen insbesondere rein softwareseitig umgesetzt.

In einer vorteilhaften Weiterbildung wird zusätzlich zur absoluten Latenz noch eine Interaktionslatenz bestimmt, welche der bisher beschriebenen absoluten Latenz insbesondere vorausgeht. Die Interaktionslatenz beschreibt die Dauer, die benötigt wird, damit eine Eingabe des Anwenders am Client am Server zu einer Veränderung der Benutzeroberfläche führt.

Bei mehreren Clients ergeben sich für die unterschiedlichen Datenströme unter Umständen auch unterschiedliche Latenzen. In einer vorteilhaften Ausgestaltung wertet der Proxy die absoluten Latenzen mehrerer Clients bezüglich eines Servers aus und bestimmt mittels eines Maximalvergleichs die größte absolute Latenz, welche dann an den Server übertragen wird, sodass dieser geeignet reagieren kann, z.B. falls die größte absolute Latenz einen Schwellwert überschreitet.

Vorzugsweise weisen der Server und der Client jeweils eine Echtzeituhr, kurz RTC (real time clock) auf, zur Bestimmung der Startzeit und der Endzeit. In einer geeigneten Ausgestaltung hierzu initialisiert der Server beim Verbindungsaufbau einen monoton aufwärts zählenden Zähler (monotonic clock), welcher insbesondere auch zur Erzeugung der Zeitstempel verwendet wird. Dadurch ist eine virtuelle Uhr realisiert, mit welcher sich der Proxy beim Verbindungsaufbau und insbesondere auch im weiteren Verlauf der Verbindung wiederkehrend synchronisiert. Daraus ermittelt der Proxy eine relative Abweichung seiner Zeit zur Zeit des Servers. Entsprechendes gilt für den Client. Auf eine tatsächliche Synchronisierung der Echtzeituhren, z.B. wie bei NTP (network time protocol), wird aber vorzugsweise verzichtet.

Die jeweilige relative Abweichung wird zweckmäßigerweise über mehrere Messungen gemittelt, sodass die Bestimmung insgesamt robuster ist. Dadurch wird außerdem kurzzeitigen Störungen der Netzwerke vorteilhaft entgegengewirkt, z.B. asymmetrischen Verzögerungen, welche bei der Bestimmung der absoluten Latenz zu Fehlern führen könnten. Mit anderen Worten: es ist eine zyklische Zeitsynchronisierung zwischen dem Server, dem Proxy und dem Client realisiert, bei welcher die Echtzeituhren selbst nicht synchronisiert werden, sondern jeder dieser Kommunikationsknotenpunkte seine eigene, lokale Zeit behält, bei welcher aber der Proxy und der Client deren Zeit zyklisch an die Zeit des Servers angleichen, nämlich durch Ermittlung der relativen Abweichung von der virtuellen Uhr des Servers. Insgesamt ist die beschriebene Synchronisation damit vorteilhaft unabhängig von tatsächlichen Uhren auf den beteiligten Geräten und somit insbesondere auch robust gegenüber Zeitumstellungen. Durch das wiederkehrende Angleichen ist die beschriebene Synchronisation vorteilhaft auch robust gegen eine relative Drift der Uhren zueinander. Diese Synchronisation ist insbesondere durch das Übertragungsprotokoll definiert und wird beim Ausführgen des Verfahrens entsprechend durchgeführt.

Vorzugsweise wird die absolute Latenz oder zumindest die Endzeit am Client in einer Rückantwort an den Server übermittelt, welcher daraus in Verbindung mit einer Ankunftszeit der Rückantwort am Server eine Umlaufzeit für die Übertragung zwischen Server und Client berechnet. Die Umlaufzeit ergibt sich insbesondere als Summe aus der absoluten Latenz und derjenigen Zeit, welche benötigt wird, bis die Rückmeldung des Clients den Server erreicht. Bei mehreren Clients ergibt sich die Umlaufzeit insbesondere aus der größten von mehreren absoluten Latenzen, also als Umlaufzeit zwischen dem Server und dem langsamsten Client. Der Server verwertet die Umlaufzeit dann insbesondere, um aus dieser weitere Informationen bezüglich der Bandbreite der Übertragung abzuleiten.

Zusätzlich zur Bestimmung der absoluten Latenz kann die Latenz als relative Latenz zwischen zwei aufeinanderfolgenden Bildern bestimmt werden. Die relative Latenz ergibt sich also z.B. als Differenz der Endzeiten, Empfangszeiten oder Ankunftszeiten zweier aufeinanderfolgender Bilder am Client. Die relative Latenz wird auch kurz als IFL (inter frame latency) bezeichnet. Die relative Latenz wird clientseitig, d.h. vom Client selbst, gemessen und vorzugsweise vom Client an den Server zurückgemeldet, insbesondere nach jedem empfangenen Bild. Im Gegensatz zur absoluten Latenz lässt sich die relative Latenz rein clientseitig bestimmten, also ohne zusätzliche Informationen vom Server.

Der Client erkennt auf Basis der relativen Latenz insbesondere, ob regelmäßig eine Aktualisierung der Anzeige erfolgt oder ob die Übertragung eingefroren oder unterbrochen ist, z.B. aufgrund einer langsamen oder unterbrochenen Verbindung. Vorzugsweise wird in diesem Zusammenhang auch eine Übertragungsgeschwindigkeit zwischen Server und Client ermittelt um eine Übermittlung über ein verlangsamtes Netzwerk zu vermeiden und in einem solchen Fall den Anwender entsprechend darauf hinzuweisen, dass die Übertragungsgeschwindigkeit zu gering ist, um eine ausreichende Sicherheit zu gewährleisten. Ein verlangsamtes Netzwerk ist z.B. ein Drahtlosnetzwerk mit temporär verringerter Übertragungsleistung oder ein kabelgebundenes Netzwerk mit beschädigtem Kabel.

Zusätzlich kann der Client die Latenz bestimmen, diese mit wenigstens einem Schwellwert vergleichen und einen Hinweis ausgeben, falls die Latenz den Schwellwert überschreitet. Der Schwellwert ist geeigneterweise einstellbar und z.B. in einem Speicher des Clients hinterlegt. Als Hinweis wird beispielsweise eine halbtransparente Überblendung der Benutzeroberfläche oder ein Text ausgegeben oder eine Kombination hiervon, welche bzw. welcher darauf hinweist, dass die Latenz den Schwellwert überschritten hat und die angezeigte Benutzeroberfläche daher möglicherweise nicht mehr aktuell ist. Zweckmäßigerweise ist der Hinweis farbcodiert, z.B. ampelartig derart, dass bei akzeptabler Latenz ein grüner Hinweis ausgegeben wird, bei geringer werdendem Abstand zum Schwellwert ein gelber Hinweise und bei Überschreiten des Schwellwerts ein roter Hinweis.

Zusätzlich kann der Client die Latenz bestimmen, diese mit wenigstens einem Schwellwert vergleiche und die Benutzeroberfläche des Servers auf dem Client sperren, falls die Latenz den Schwellwert überschreitet. Dadurch wird eine Eingabe über den Client blockiert. Entsprechende Eingabegeräte werden hierzu ggf. blockiert, d.h. deren Eingaben nicht an den Server weitergeleitet. Dadurch wird eine mögliche Falscheingabe z.B. aufgrund einer sich zwischenzeitlich geänderten aber aufgrund der Latenz nicht angezeigten Zuordnung von Eingabeelementen vorteilhaft verhindert. Auch laufende Eingaben werden zweckmäßigerweise unterbrochen, falls der Schwellwert überschritten wird.

Der Schwellwert ist allgemein entweder vorbestimmt und z.B. in einem Speicher des Clients hinterlegt oder wird vom Server dynamisch festgelegt, z.B. bei einem Verbindungsaufbau zwischen Server und Client. Beide Varianten sind vorteilhaft und auch kombinierbar. Der dynamischen Festlegung liegt insbesondere der Gedanke zugrunde, dass das Master-Gerät zunächst keine Kenntnis davon hat, welche Latenzen für einen Server noch akzeptabel sind, dies wird vielmehr durch den Server selbst vorgegeben, da der Hersteller des Servers die konkreten Risiken kennt und eine entsprechende Risikomitigationsstrategie im Server implementiert. Der Schwellwert wird dann vom Server vorgegeben und mittels des Übertragungsprotokolls dem Client übermittelt z.B. während der Laufzeit, also bei bestehender Verbindung.

Vorteilhaft ist allgemein auch ein abgestaffelter Schwellwert, d.h. der Schwellwert umfasst dann mehrere Stufen für die Latenz, bei deren jeweiligem Überschreiten ein anderer, entsprechender Hinweis ausgegeben wird, z.B. der oben beschriebene ampelartige Hinweis, oder eine andere, entsprechende Aktion ausgeführt wird oder eine Kombination hiervon. Mit einem abgestaffelten Schwellwert, genauer gesagt also mit mehreren Schwellwerten, ist eine besonders feine Abstimmung des Verhaltens des Servers, des Proxys oder des Clients mittels gestaffelten Reaktionen realisiert.

Bei einer Ausgestaltung mit mehreren Clients wird die Latenz vorzugsweise individuell für jeden Client und somit individuell für jeden Datenstrom erkannt, d.h. für jede einzelne Verbindung zwischen dem Server und dem jeweiligen Client. Dasselbe gilt analog bei einer Ausgestaltung mit mehreren Servern.

Zweckmäßig ist auch eine Ausgestaltung, bei welcher alternativ oder zusätzlich zum Client der Proxy die Latenz bestimmt, da sich der Proxy besonders bei mehreren Clients zur Steuerung der diversen Datenströme mit ggf. unterschiedlichen Anforderungen eignet und hierbei zweckmäßigerweise die jeweilige Latenz berücksichtigt.

Geeignet ist auch eine Ausgestaltung, bei welcher eine der Komponenten die Latenz bestimmt und an eine andere der Komponenten sendet, insbesondere als Teil eines Pakets, da ja die Latenz an ein bestimmtes Paket gekoppelt ist. In einer bevorzugten Ausgestaltung bestimmt der Client die Latenz und übermittelt diese an den Server in einer Rückantwort, um dem Server eine Reaktion auf die Latenz zu ermöglichen und z.B. um auf Basis einer Risikoabwägung die Ausgabe der Benutzeroberfläche zu ändern und an die Latenz anzupassen. Die Reaktion des Servers ist insbesondere herstellerseitig definiert. Analoges gilt für die Bestimmung der Latenz durch den Proxy und eine entsprechende Weiterleitung an den Server.

Der Datenstrom, welcher von einem Server erzeugt wird, ist für den Client möglicherweise zu umfangreich. Mit anderen Worten: grundsätzlich besteht die Gefahr, dass der Client eine Bildwiederholrate des Servers nicht unterstützt, sondern eine geringere Bildwiederholrate aufweist, sodass der Client durch die Menge an Bildern überladen wird, woraus sich entsprechend Fehldarstellungen der Benutzeroberfläche ergeben können. Auch ein Volllaufen eines Empfangspuffers des Clients ist unter Umständen möglich, wodurch sich eine entsprechende, nachteilige Latenz aufbaut. Um eine solche Überladung oder ein Volllaufen eines im Vergleich zum Server leistungsschwachen Clients zu vermeiden, wird zweckmäßigerweise eine Bandbreitensteuerung vorgenommen, bei welcher die Größe des Datenstroms zwischen Server und Client durch den Proxy gesteuert werden. Die Bildwiederholrate des Servers wird dabei durch den Proxy im Rahmen der Bandbreitensteuerung vorzugsweise an den Client angepasst, um diesen zu entlasten.

Die Bandbreitensteuerung ist auch vorteilhaft vor dem Hintergrund, dass zusätzlich zu den Bilddaten der Benutzeroberfläche regelmäßig auch andere Daten übermittelt werden, z.B. Steuerbefehle. Um das interne und das externe Netzwerk besonders kostengünstig zu gestalten, unterstützen diese insbesondere keine Qualitätssicherung, kurz QoS (quality of service), wie dies z.B. bei einem Echtzeit-Netzwerk der Fall wäre. Eine Bandbreitensteuerung durch den Proxy verhindert nun vorteilhafterweise eine Störung der Übertragung anderer Daten, indem die Übermittlung von Bilddaten zugunsten anderer Daten begrenzt wird, sodass letztere besonders zuverlässig übertragen werden.

Nachfolgend werden vier bevorzugte Konzepte zur Bandbreitensteuerung, d.h. speziell zur Begrenzung der Bildwiederholrate beschrieben, nämlich eine statische Bandbreitensteuerung, eine dynamische Bandbreitensteuerung, eine Bandbreitensteuerung durch Deaktivierung nicht benötigter Datenströme und eine Bandbreitensteuerung durch eine Kodierung der Bilder durch den Proxy. Die dynamische Bandbreitensteuerung ist genauer gesagt eine Bandbreitenregelung, nachfolgend wird jedoch auch hierfür der allgemeinere Begriff der Bandbreitensteuerung verwendet. Vorteilhaft ist auch eine Kombination der statischen und der dynamischen Konzepte, d.h. deren parallele Anwendung. Eine besonders vorteilhafte Kombination ist derart, dass die dynamische Begrenzung subsidiär zur statischen Begrenzung verwendet wird, d.h. wenn zur statischen Begrenzung keine bestimmte Bildwiederholrate für den betreffenden Client im Proxy gespeichert ist und eine statische Begrenzung entsprechend nicht möglich ist. Die Bandbreitensteuerung durch Deaktivierung nicht benötigter Datenströme ist besonders geeignet bei einer Ausgestaltung mit mehreren Servern.

In einer geeigneten Ausgestaltung versendet der Server die Bilder mit einer Bildwiederholrate, welche vom Proxy auf eine maximale Bandbreite insbesondere des internen Netzwerks begrenzt wird, indem der Proxy innerhalb eines gegebenen Zeitabschnitts lediglich solange Bilder an den Client weiterleitet, bis die maximale Bandbreite erreicht ist, und alle nachfolgenden Bilder innerhalb des Zeitabschnitts nicht an den Client weiterleitet. Im Ergebnis begrenzt der Proxy beim Durchleiten an den Client die vom Server bereitgestellte Bildwiederholrate und erzeugt eine neue, angepasste und insbesondere verringerte Bildwiederholrate. Dadurch ist eine statische Begrenzung realisiert, nämlich statisch im Sinne der Bandbreite, wohingegen die Bildanzahl pro Zeiteinheit durchaus variieren kann. Dabei erfolgt sozusagen eine rollierende Summenbildung der Bilddatenmenge, welche übertragen wird, und ein Auslassen von Bildern bei Überschreiten einer Obergrenze für die Datenmenge, d.h. bei Erreichen der maximalen Bandbreite. Beispielsweise wird bei einer Bildwiederholrate des Servers von 30 Bildern/s über einen Zeitabschnitt von 1 s die Bandbreite bestimmt, d.h. die Bandbreite der letzten 30 Bilder errechnet. Ausgehend vom ersten Bild dieser 30 Bilder werden dann so viele Bilder weitergeleitet, bis die maximale Bandbreite erreicht ist, alle ggf. übrigen Bilder werden verworfen. Bei 30 Bildern/s steht nach 33 ms ein neues Bild bereit und der Vorgang wird entsprechend mit verschobenen Betrachtungsrahmen wiederholt, sodass das älteste Bild aus der Summierung herausfällt. Die verworfenen Bilder werden vom Proxy nicht an den Client weitergeleitet. Falls ein Bild verworfen wird, wird dies vom Proxy in einer Rückantwort an den Server vermerkt, sodass der Server je nach Ausgestaltung entsprechend reagieren kann.

Die maximale Bandbreite gibt die maximale Datenmenge pro Zeitabschnitt, d.h. Zeiteinheit an, welche zum Client weitergeleitet wird. Die maximale Bandbreite beträgt z.B. 100 MBit/s. Der statischen Bandbreitensteuerung liegt insbesondere die Überlegung zugrunde, dass die einzelnen Bilder aufgrund einer Komprimierung unter Umständen unterschiedlich groß sein können und daher je nach Komprimierung eine variierende Bandbreite beanspruchen. Bei stärker komprimierten Bildern werden dann mehr Bilder pro Zeiteinheit an den Client weitergeleitet als bei weniger stark komprimierten Bildern, um insgesamt die maximale Bandbreite einzuhalten. Durch die statische Bandbreitensteuerung wird nicht nur die Rechenkapazität des Clients geschont, sondern vorteilhaft auch das interne Netzwerk, sodass parallel zu den Bilddaten noch andere Daten, z.B. Steuerbefehle gefahrlos übertragen werden und das interne Netzwerk nicht Gefahr läuft, von einer zu großen Menge an Bilddaten überlaufen zu werden. Beispielsweise wird die Bandbreite für Bilddaten durch die Bandbreitensteuerung auf 50 % der Bandbreite des internen Netzwerks begrenzt.

Geeignet ist auch eine Ausgestaltung, bei welcher die Bildwiederholrate des Servers vom Proxy nicht oder nicht ausschließlich auf eine maximale Bandbreite reduziert wird, sondern auf eine maximale Bildwiederholrate des Clients. Die maximale Bildwiederholrate entspricht einer maximalen Bildanzahl innerhalb eines vorgegebenen Zeitabschnitts. Bei der statischen Begrenzung werden die ersten Bilder innerhalb dieses Zeitraums übertragen, bis die maximale Bildanzahl für diesen Zeitraum erreicht ist und alle nachfolgenden Bilder innerhalb des Zeitraums werden verworfen, d.h. nicht übertragen. Beispielsweise beträgt die maximale Bildwiederholrate des Clients 30 Bilder/s und der Server sendet Bilddaten mit einer Bildwiederholrate von 40 Bilder/s, dann werden pro Sekunde lediglich die ersten 30 Bilder übertragen und die verbleibenden 10 Bilder nicht. Dieses Konzept ist besonders geeignet einerseits zur Vermeidung einer Überforderung des internen Netzwerks und andererseits auch für Clients mit besonders begrenzter Rechenkapazität, beispielsweise bei einem Monitor des Hauptgeräts eines mehrteiligen C-Bogens. Bei solchen Clients ist dann durch die Begrenzung auf eine maximale Bildwiederholrate zu jedem Zeitpunkt sichergestellt, dass die empfangenen Bilddaten nicht die Rechenkapazität des Clients überfordern.

Die statische Begrenzung wird zweckmäßigerweise clientspezifisch durchgeführt, d.h. für jeden Client separat. Zweckmäßigerweise ist die maximale Bildwiederholrate einstellbar und wird vom Proxy je nach Client geeignet eingestellt. Die maximale Bildwiederholrate ist in einem Speicher des Proxys hinterlegt.

Ebenfalls gemäß der Erfindung leitet der Proxy die Bilder vom Server mit einer Bildwiederholrate weiter, welche dynamisch angepasst wird, indem eine Umlaufzeit zwischen dem Server und dem Client ermittelt wird und abhängig von der Umlaufzeit lediglich eine Teilmenge an Bildern an den Client weitergeleitet wird. Dadurch ist eine dynamische Begrenzung realisiert, bei welcher die Bildwiederholrate dynamisch an eine insbesondere variierende Rechenkapazität des Clients angepasst wird. Die Umlaufzeit, kurz RTT (round trip time) wurde oben bereits beschrieben und entspricht der Summe aus derjenigen Zeit, welche die Übertragung eines Bildes vom Server zum Client benötigt und derjenigen Zeit, welche eine Rückantwort des Clients hierauf zum Server benötigt. Falls die Umlaufzeit einen bestimmten oberen Schwellwert überschreitet wird die Bildwiederholrate reduziert, falls dagegen die Umlaufzeit einen anderen, unteren Schwellwert unterschreitet, wird die Bildwiederholrate erhöht.

Die Umlaufzeit dient also als eine Rückkopplung für eine Regelung der Bandbreite. Eine zunehmende Umlaufzeit signalisiert insbesondere das Volllaufen eines Eingangspuffers des Clients, sodass in diesem Fall die Bildwiederholrate zweckmäßig reduziert wird. Umgekehrt signalisiert eine sinkende Umlaufzeit eine freie Kapazität des Clients. Entsprechendes gilt analog für die verfügbare Bandbreite des internen Netzwerks. Bei diesem Konzept ist demnach insgesamt eine optimale Anpassung der Bildwiederholrate an die momentane Arbeitsbelastung und damit die momentan freie Rechenkapazität des Clients einerseits sowie andererseits an die freie Bandbreite des internen Netzwerks gewährleistet, sodass insgesamt eine möglichst hohe Bildwiederholrate erzielt wird. Dies ist besonders geeignet bei einem insbesondere eigenständigen Client mit besonders hoher Rechenkapazität, beispielsweise einem Monitor des Nebengeräts eines mehrteiligen C-Bogens.

Die Umlaufzeit wird in einer geeigneten Ausgestaltung vom Proxy bestimmt, welcher hierzu entsprechende Daten vom Server und vom Client erhält. Alternativ wird die Umlaufzeit vom Client oder vom Server bestimmt und an den Proxy übermittelt. Beispielsweise sind eine Startzeit am Server und eine Endzeit am Client gemeinsam mit dem Bild oder der Rückantwort in einem Paket enthalten und werden vom Proxy ausgewertet.

In einer zweckmäßigen Ausgestaltung mit dynamischer Begrenzung ist die Teilmenge an Bildern, welche weitergeleitet wird durch einen einstellbaren Teiler bestimmt, welcher eine Anzahl aufeinanderfolgender Bilder angibt, von welchen eines weitergeleitet wird und alle übrigen verworfen werden. Die Bildwiederholrate wird nun abhängig von der Umlaufzeit angepasst, indem der Teiler um 1 erhöht wird, falls die Umlaufzeit einen oberen Schwellwert überschreitet, und um 1 verringert wird, falls die Umlaufzeit einen unteren Schwellwert unterschreitet.

Standardmäßig beträgt der Teiler vorzugsweise 1, sodass jedes Bild des Servers an den Client übertragen wird. Überschreitet die Umlaufzeit den oberen Schwellwert, wird der Teiler in ganzzahligen Schritten auf einen Wert X erhöht, sodass nur noch jedes X-te Bild an den Client weitergeleitet wird und die übrigen Bilder verworfen werden. Umgekehrt wird der Teiler in ganzzahligen Schritten verringert, falls der untere Schwellwert von der Umlaufzeit unterschritten wird. In einer geeigneten Ausgestaltung wird der Teiler solange schrittweise erhöht, wie die Umlaufzeit oberhalb des oberen Schwellwerts liegt und konstant gehalten, wenn die Umlaufzeit unterhalb des oberen Schwellwerts liegt. Analoges gilt für den unteren Schwellwert und die Reduzierung des Teilers, wobei der Teiler als niedrigsten Wert 1 annehmen kann, in welchem Fall die Bildwiederholrate des Servers vollständig vom Client unterstütz wird.

Unabhängig davon, ob eine statische oder dynamische Begrenzung erfolgt, werden diejenigen Bilder, welche vom Proxy nicht an den Client weitergeleitet werden, in einer Rückantwort an den Server zweckmäßigerweise als verworfen markiert.

Bei mehreren Servern, auch bei mehreren virtuellen Servern, welche gemeinsam auf einem (als Zahlwort) Client dargestellt werden, d.h. allgemein bei mehreren Datenströmen für einen Client, ist eine Bandbreitensteuerung vorteilhaft, bei welcher nicht benötigte Datenströme deaktiviert werden, sodass weiter Bandbreite eingespart wird und das interne Netzwerk und der Client entlastet werden. In einer geeigneten Ausgestaltung hiervon wird auf dem Client zu einem gegebenen Zeitpunkt lediglich eine Teilanzahl von mehreren Datenströmen mehrerer Server angezeigt und die übrigen Datenströme werden pausiert, sodass deren Bilder nicht zum Client übertragen werden, sodass insgesamt eine Bandbreitenreduzierung erzielt wird. Somit werden gerade solche Datenströme deaktiviert, welche vom Client momentan nicht angezeigt werden, z.B. da diese gerade nicht für den Anwender von Bedeutung sind oder vom Anwender nicht angefordert worden sind oder da deren Bereiche zur Anzeige von anderen Bereichen verdeckt sind. Bilder, welche nicht benötigt werden, werden somit nicht angezeigt. Der Client meldet hierzu an den Server, welche Datenströme tatsächlich angezeigt werden und alle nicht angezeigten Datenströme werden vom Server deaktiviert, d.h. die Übertragung von Bildern dieser Datenströme wird pausiert.

In einer weiteren bevorzugten Ausgestaltung transkodiert der Proxy die Bilder, welche vom Server zum Client weitergeleitet werden, derart, dass deren Größe reduziert wird, um weniger Bandbreite im internen Netzwerk zu beanspruchen und dieses entsprechend zu entlasten. Sofern die Bilder bereits vom Server komprimiert wurden, führt der Proxy eine weitere Reduktion der Datenmenge durch. Sofern die Bilder vom Server unkomprimiert sind, werden diese nun durch den Proxy komprimiert.

Für die Verbindung vom Server über den Proxy zum Client wird durch den Proxy oder den Client oder beide ein Verbindungsverlust vorzugsweise mittels einer Aktivitätsprüfung erkannt. Dabei wird vom Proxy oder vom Client oder von beiden die Zeit gemessen, welche seit Erhalt des zuletzt übermittelten Bildes vergangen ist. Falls diese Zeit einen vorgegebenen Schwellwert von z.B. 1 s überschreitet, wird ein Verbindungsverlust festgestellt. Bei einer pausierten Verbindung, z.B. wie oben beschrieben, wird jedoch regelmäßig kein Bild mehr übertragen, obwohl die Verbindung an sich noch besteht und nutzbar ist. Um in diesem Fall zu verhindern, dass fälschlicherweise ein Verbindungsverlust festgestellt wird, wird bei einer solchen pausierten Verbindung anstelle eines tatsächlichen Bildes regelmäßig ein Platzhalter übertragen, welcher möglichst wenig Bandbreite beansprucht, z.B. ein einzelnes Pixel, und die Verbindung dadurch aktiv gehalten. Das Aktivhalten der Verbindung insbesondere ggf. mittels eines Platzhalters wird auch als "heartbeat" bezeichnet. Umgekehrt wird für die Verbindung vom Client über den Proxy zum Server von diesem ein Verbindungsverlust ebenfalls mittels einer entsprechenden Aktivitätsprüfung erkannt. Hierzu wird vom Client oder vom Proxy oder von beiden eine Rückantwort an den Server gesendet, sobald ein Paket des Servers vom Proxy oder vom Client empfangen worden ist. Der Server misst dann die Zeit bis zum Erhalt der Rückantwort und stellt einen Verbindungsverlust fest, falls diese Zeit einen vorgegebenen Schwellwert von z.B. 1 s überschreitet. Die Reaktion des Servers auf einen Verbindungsverlust ist insbesondere herstellseitig definiert.

Die Begrenzung der Bandbreite stellt insbesondere ein adaptives Bandbreitenmanagement dar. Da je nach Rechenkapazität des Clients Bilder ausgelassen werden, werden weniger Daten versendet und das interne Netzwerk wird weniger belastet. Dies ist auch vorteilhaft, da die Übertragung der Benutzeroberfläche insbesondere parallel zu weiterem und möglicherweise zeitkritischem Datenverkehr existiert. Um diesen Datenverkehr nicht zu kompromittieren wird die Bandbreitennutzung bedarfsweise reduziert. Ein kostenintensives Echtzeit-Netzwerk oder ebenfalls kostenintensive Feldbus-Hardware zur Steuerung der diversen Ströme an Bildern und anderen Daten wird nicht benötigt, vorzugsweise wird auf solche sogar verzichtet.

Insgesamt bieten das Verfahren und das dabei angewendete Übertragungsprotokoll diverse Möglichkeiten zur Überwachung der Übertragung zwischen einem Server und einem Client. Aus dem bereits Gesagten ergibt sich insbesondere die Möglichkeit eine Übertragungsgeschwindigkeit für Daten zu ermitteln, eine Datenübergabe zu überwachen, eine Aktivitätsprüfung durchzuführen, einen Verbindungsverlust festzustellen, eine Umlaufzeit zu messen, die Latenz bei der Übertragung von Daten dient-, proxy- oder serverseitig zu messen, einen Unterschied zwischen Echtzeituhren als Taktgeber für virtuelle Uhren in den Servern zu messen und den Proxy, den Server und den Client zu synchronisieren und wiederkehrende Aktualisierungen zu überwachen. Einem Server stehen somit zur Reaktion je nach Ausgestaltung eine oder mehrere der folgenden Informationen zur Verfügung: die Bandbreite des externen Netzwerks; welcher von mehreren Clients der langsamste Client ist; das Ergebnis einer Prüfung einer Aktivität, auch als "heartbeat" bezeichnet; eine Umlaufzeit zwischen dem Server und einem jeweiligen Client; eine relative oder absolute Latenz oder beides; eine Information, ob und insbesondere auch welche Bilder bei der Übertragung zu einem jeweiligen Client verworfen wurden; eine Information, ob ein Bild verspätet von einem Client empfangen wurde, z.B. weil ein Schwellwert für die relative Latenz überschritten wurde oder sich aus einem anderen Grund eine Verlangsamung ergab. Gemeinsam ist allen diesen Möglichkeiten und Informationen der Vorteil, dass durch deren Anwendung bzw. Verwendung ein funktionales Risiko allgemein einer verzögerten Übertragung und speziell einer verzögerten Anzeige einer Benutzeroberfläche eines Servers auf einem Client deutlich abgeschwächt oder sogar eliminiert wird. Daraus ergibt sich eine verbesserte funktionale Sicherheit bei der Nutzung eines jeweiligen Servers.

Zweckmäßigerweise wird gemäß dem Übertragungsprotokoll bei dem Verfahren auch die Bildintegrität überwacht, d.h. die Integrität der einzelnen Bilder. Hierzu wird als Teil eines jeweiligen Pakets eine Prüfsumme mitgesendet, z.B. CRC32, welche vom Client geprüft wird. Stellt der Client eine Abweichung von der Prüfsumme fest, wird das Bild nicht angezeigt und in der entsprechenden Rückantwort an den Server als defekt gekennzeichnet. Diese Integritätsprüfung der Bilddaten ist speziell bei UDP als Transportschicht vorteilhaft.

Die Benutzeroberfläche, welche vom Server ausgegeben wird, ist insbesondere von dessen Hersteller vorgegeben. Der Client skaliert die Benutzeroberfläche geeigneterweise je nach Bedarf auf eine Anzeigefläche, welche vom Client oder vom Proxy hierfür bestimmt worden ist und skaliert auch eine eventuelle Eingabefläche entsprechend. Geeignet ist auch eine Ausgestaltung, bei welcher der Server selbst seine Benutzeroberfläche auf eine Anzeigefläche des Clients skaliert. Vorzugsweise unterstützt der Server bereits die vom Client verwendete Bildauflösung, z.B. 1280 x 720 Pixel, um eine möglichst originalgetreue Abbildung am Client zu erhalten.

Geeigneterweise ist der Client dazu ausgebildet, mehrere Benutzeroberflächen gemeinsam, insbesondere nebeneinander anzuzeigen und dadurch eine parallele Anzeige zu realisieren. Besonders zweckmäßig ist eine Ausgestaltung mit einer sogenannten hybriden Anzeige. Bei dieser Ausgestaltung ist eine Anzeigefläche des Clients in zwei Anzeigebereiche unterteilt, nämlich einen ersten Bereich für die Benutzeroberfläche des Master-Geräts, also zur Eigenanzeige, und einen zweiten Bereich für die Benutzeroberfläche eines oder mehrerer Server, also zur Fremdanzeige. Die Benutzeroberflächen der Server und des Master-Geräts bilden dann sozusagen Untereinheiten einer Master-GUI und sind insgesamt von einem zentralen Ort aus nutzbar. Zweckmäßigerweise ist der Client derart ausgebildet, dass dieser die Benutzeroberfläche eines jeweiligen Servers je nach Bedarf in dem zweiten Bereich anzeigt, d.h. genau dann, wenn eine Interaktion des Anwenders mit dem Server erfolgen soll. Zweckmäßigerweise ist der zweite Bereich umschaltbar zwischen der Eigenanzeige und der Fremdanzeige, sodass die gesamte Anzeigefläche des Clients zur Interaktion mit dem Master-Gerät nutzbar ist und bedarfsweise zur Interaktion mit einem Server umgeschaltet wird. Diese Ausgestaltung wird bei einem mehrteiligen Master-Gerät vorzugsweise bei einem Monitor des Hauptgeräts verwendet.

Die hybride Anzeige ist besonders vorteilhaft bei Verwendung eines bildgebenden Medizingeräts als Master-Gerät, da hier unter Umständen aus regulatorischen Gründen permanent bestimmte Informationen angezeigt werden müssen, z.B. hinsichtlich einer Strahlenquelle. Solche Informationen sind dann z.B. der Aktivitätsstatus der Strahlenquelle, deren Spannung, Strom, Dosis und Strahlzeit. Diese Informationen werden dann permanent im ersten Bereich angezeigt, während im zweiten Bereich je nach Bedarf wechselnd andere Informationen angezeigt werden. Dasselbe gilt analog für ggf. permanent anzuzeigende Informationen eines Servers. Bei einem mehrteiligen Master-Gerät wird die hybride Anzeige vorzugsweise bei den Monitoren des Nebengeräts verwendet.

Zweckmäßigerweise ist ein Client mit hybrider Anzeige derart ausgebildet, dass Anfragen des Master-Geräts an den Anwender in Form von Dialogfenstern im zweiten Bereich angezeigt werden und dann die dortige Fremdanzeige überlagern, zumindest temporär, d.h. bis die notwendige Eingabe erfolgt ist. Dadurch wird für die Interaktion mit dem Master-Gerät eine vergrößerte Anzeigefläche bereitgestellt, sodass der erste Bereich zur Eigenanzeige entsprechend platzsparend dimensioniert werden kann und entsprechend auch deutlich geringer dimensioniert ist als der zweite Bereich zur Fremdanzeige. Insbesondere enthält der erste Bereich dann überwiegend oder lediglich die oben beschriebenen permanent anzuzeigenden Informationen, während optionale Interaktionen lediglich bei Bedarf angezeigt werden und dann im zweiten Bereich erfolgen.

Der Client ist zweckmäßigerweise mit einer Komfortfunktion derart ausgestattet, dass unnötige Interaktionen des Anwenders möglichst reduziert werden. Dies betrifft insbesondere diejenigen Fälle, in welchen eine Interaktion mit einem Server zugleich auch eine bestimmte Interaktion mit dem Master-Gerät erfordert. In diesem Fall werden im Rahmen der Komfortfunktion solche Anzeigefelder und Eingabefelder der Benutzeroberfläche des Master-Geräts, welche bei der Interaktion mit einem Server benötigt werden, gemeinsam mit der Benutzeroberfläche des Servers angezeigt und zweckmäßigerweise sogar in diese integriert. Die nötigen Ausgabe- und Eingabefelder des Master-Geräts werden insbesondere vom Server angefragt.

Ein weiterer Vorteil der Erfindung ist insbesondere, dass mehrere Server mit mehreren Clients verbindbar sind und dadurch mehrere Geräte gebündelt über ein einzelnes Master-Gerät mit mehreren Clients zur Anzeige der Benutzeroberflächen steuerbar sind. Dabei werden einerseits die Benutzeroberflächen mehrerer Server gleichzeitig und unabhängig voneinander über die Clients dargestellt und andererseits können auch die Eingaben für die Server gleichzeitig und unabhängig voneinander von den Clients erfolgen. Der Proxy vermittelt dabei vorzugsweise derart zwischen mehreren Clients, dass bei einer Eingabe über einen der Clients eine zusätzliche Eingabe über die anderen Clients gesperrt ist, sodass ein Server zu einem gegebenen Zeitpunkt also immer lediglich über einen Client steuerbar ist. Eine Übergabe der Steuerung von einem Client auf einen anderen Client wird dabei zweckmäßigerweise unterbunden.

Bei mehreren Servern ist ein jeweiliger Server insbesondere ein für sich genommen eigenständiges Medizingerät mit wenigstens einer bestimmten klinischen Funktion. Ein jeweiliger Server weist typischerweise ein eigenes Ausgabegerät auf, zur Ausgabe von Informationen, sowie ein Eingabegerät, zur Steuerung. Mehrere Server sind entsprechend physisch voneinander unterscheidbar und stellen entsprechend mehrere, separate Medizingeräte dar. Alternativ oder zusätzlich wird ein Server mit mehreren Instanzen betrieben, sodass der Server sozusagen mehrere virtuelle Geräte aufweist, mit entsprechend vielen Benutzeroberflächen, welche dann simultan mittels der Clients angezeigt werden und dort zur Steuerung der virtuellen Geräte dienen.

Bei mehreren Clients ist ein jeweiliger Client zur Anzeige wenigstens einer Benutzeroberfläche und zur Interaktion mit dieser ausgebildet. Hierzu weist der Client ein Ausgabegerät auf, um die Benutzeroberfläche anzuzeigen und somit Informationen auszugeben. Das Ausgabegerät ist bevorzugterweise ein Monitor. Zur Interaktion weist der Client weiter auch ein Eingabegerät auf, um Steuerbefehle einzugeben. Das Eingabegerät ist beispielsweise eine Tastatur oder Maus. Besonders bevorzugt ist eine Ausgestaltung, bei welcher der Client einen Touchscreen aufweist, welcher zugleich Ausgabe- und Eingabegerät ist und eine direkte Interaktion mit der Benutzeroberfläche ermöglicht. Der Touchscreen ist zur Singletouch- oder Multitoucheingabe ausgebildet.

Die Server, der Proxy und die Clients, stellen jeweils einen Kommunikationsknotenpunkt eines Gesamtnetzwerks dar, welches auch das interne und das externe Netzwerk enthält. Jeder Kommunikationsknotenpunkt ist insbesondere zur eigenständigen Durchführung des Übertragungsprotokolls ausgebildet und führt insbesondere auch eine Fehlerkorrektur oder einen Neustart nach einem Fehler oder Verbindungsfehler selbstständig durch, d.h. unabhängig von den übrigen Kommunikationsknotenpunkten. Zweckmäßigerweise wird bei einem solchen Vorgang ein entsprechender Hinweis an den Anwender ausgegeben, speziell, dass in dieser Situation eine sichere Übertragung nicht gewährleistet ist.

Jeder Server stellt insbesondere seine eigenen Verbindungsparameter bereit, welche insbesondere Bildauflösung, Bildkodierung oder Bildformat oder eine Kombination hiervon umfassen, sodass in einer geeigneten Ausgestaltung mehrere Server mit unterschiedlichen Verbindungsparametern betrieben werden. Dadurch ist es möglich, unterschiedliche Geräte, insbesondere von unterschiedlichen Herstellern miteinander zu kombinieren und gemeinsam mittels des Master-Geräts zu steuern.

Zur Übertragung wird insbesondere eine verlustlose oder eine verlustbehaftete Kodierung verwendet. Je nach Anwendung und Bandbreite, welche zur Verfügung steht, sind die Bilder verlustlos oder verlustbehaftet komprimiert. Dies wird insbesondere serverseitig festgelegt, da der Server seine Anwendung prinzipbedingt kennt und daher am ehesten in der Lage ist zu entscheiden, ob eine verlustbehaftete Komprimierung zulässig und akzeptabel ist oder nicht. Optional verwendet das Übertragungsprotokoll eine Verschlüsselung sowie eine korrespondierende Authentifizierung. Das Übertragungsprotokoll und insbesondere auch der Proxy, welcher eine Schnittstelle des Master-Geräts zur Außenwelt darstellt, sind zweckmäßigerweise möglichst robust ausgelegt gegenüber üblichen Störungen des externen Netzwerks, z.B. Verzögerungen, Paketverlust, d.h. Datenverlust, Neuversendung von Daten, Hackerangriffen und dergleichen.

Für jede Verbindung eines Servers mit dem Master-Gerät ist zweckmäßigerweise ein Handshake notwendig, mittels welchem der Server signalisiert, dass dieser das spezielle Übertragungsprotokoll unterstützt. Beim Handshake wird insbesondere eine Versionsnummer des Übertragungsprotokolls ausgetauscht. Optional werden beim Handshake noch ein oder mehrere andere Parameter ausgetauscht, z.B. Identifizierungsinformationen wie eine Geräte-ID oder eine Seriennummer, Lizenzinformationen, Symbole, Grafiken oder Texte, um z.B. eine Anwendung des Servers auf dem Client durch ein spezielles Icon oder Label darzustellen.

Die Interaktion mit einem Server über einen Client ist zudem vorzugsweise abhängig vom Vorhandensein einer Nutzungslizenz, welche auf dem Server gespeichert ist und von diesem z.B. als Lizenzinformation beim Handshake übermittelt wird, um eine Übertragung der Benutzeroberfläche zu erlauben. Der Proxy erfüllt vorteilhafterweise eine Wächterfunktion derart, dass kein gefährlicher oder feindlicher Zugriff vom externen Netzwerk auf das interne Netzwerk möglich ist. Insbesondere unterstützt der Proxy auch den Handshake des Servers, speziell zur Authentifizierung und Verschlüsselung. Vorzugsweise erfolgt jegliche Kommunikation, d.h. Übertragung von Daten, zwischen Server und Client ausschließlich über den Proxy.

Nachfolgend werden Ausführungsbeispiele der Erfindung anhand einer Zeichnung näher erläutert. Darin zeigen jeweils schematisch:
- FIG 1: einen Proxy sowie mehrere Server und mehrere Clients,
- FIG 2: eine Anzeige auf einem Client,
- FIG 3: eine andere Anzeige auf einem Client,
- FIG 4: eine andere Anzeige auf einem Client,
- FIG 5: eine Bestimmung einer absoluten Latenz,
- FIG 6: eine Bestimmung einer absoluten Latenz durch einen Client,
- FIG 7: eine Bestimmung einer absoluten Latenz bei mehreren Clients,
- FIG 8: eine Bestimmung einer relativen Latenz,
- FIG 9: eine statische Bandbreitenreduktion,
- FIG 10: eine dynamische Bandbreitenreduktion.

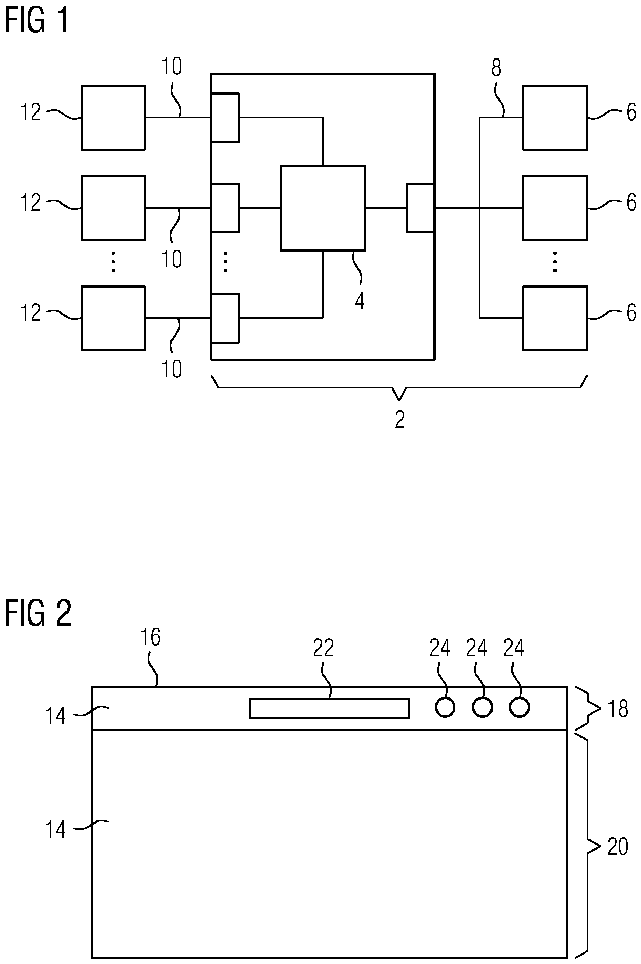

In FIG 1 ist ein Master-Gerät 2 gezeigt, welches hier ein bildgebendes Medizingerät ist, nämlich ein C-Bogen. Das Master-Gerät 2 weist einen Proxy 4 und mehrere Clients 6 auf, welche über wenigstens ein internes Netzwerk 8 miteinander verbunden sind. Der Proxy 4 bildet eine Schnittstelle des Geräts 2 nach außen und ist über wenigstens ein externes Netzwerk 10 mit mehreren Servern 12 verbunden, welche jeweils ein eigenständiges Medizingerät sind. Die Ausführungen zu dieser Anordnung gelten sinngemäß auch für nicht gezeigt Ausgestaltungen mit nur einem Server 12 oder nur einem Client 6. Das interne Netzwerk 8 ist hier beispielhaft ein TCP/IP-Netzwerk. Das externe Netzwerk 10 basiert hier beispielhaft auf einem kabelgebundenen Standard-Ethernet. In einer Variante ist wenigstens eines der Netzwerke 8, 10 kabellos. Sämtliche Daten, welche von einem Server 12 zu einem Client 6 oder umgekehrt übertragen werden, laufen durch den Proxy 4.

Die Clients 6 sind hier jeweils als Touchscreen ausgebildet, um alternativ oder in Kombination mit einer Benutzeroberfläche 14 des Master-Geräts 2 selbst auch eine oder mehrere Benutzeroberflächen 14 eines oder mehrerer Server 12 anzuzeigen. Die Ausgestaltung als Touchscreen ist nicht zwingend. In einer Variante weist ein Client 6 einen Monitor lediglich zur Anzeige auf und als Eingabegeräte z.B. eine Maus oder Tastatur. Auch eine Kombination von Touchscreen mit einem weiteren Eingabegerät ist möglich. Das Master-Gerät 2 ist hier mehrteilig ausgebildet und weist eine Haupteinheit auf, welche den Proxy 4, einen der Clients 6 sowie einen nicht dargestellten C-Arm umfasst, sowie eine Nebeneinheit, welche die übrigen Clients 6 umfasst. Die Server 12 sind ebenfalls Medizingeräte, ggf. jedoch von einem anderen Hersteller. Beispielsweise ist einer der Server 12 ein Navigationsgerät.

Vorliegend weist jeder Server 12 sowie das Master-Gerät 2 eine Benutzeroberfläche 14 auf, auch als GUI (graphical user interface) bezeichnet. Die Benutzeroberflächen 14 der Server 10 werden über den Proxy 4 auf die Clients 6 übertragen und dort angezeigt. Dadurch ist die Benutzeroberfläche 14 eines jeden Servers 12 auf jedem der Clients 6 zugleich darstellbar und umgekehrt sind analog auch mehrere Server 12 von einem einzelnen Client 6 aus steuerbar. Ein jeweiliger Client 6 ist dazu ausgebildet, mehrere Benutzeroberflächen 14 gemeinsam anzuzeigen.

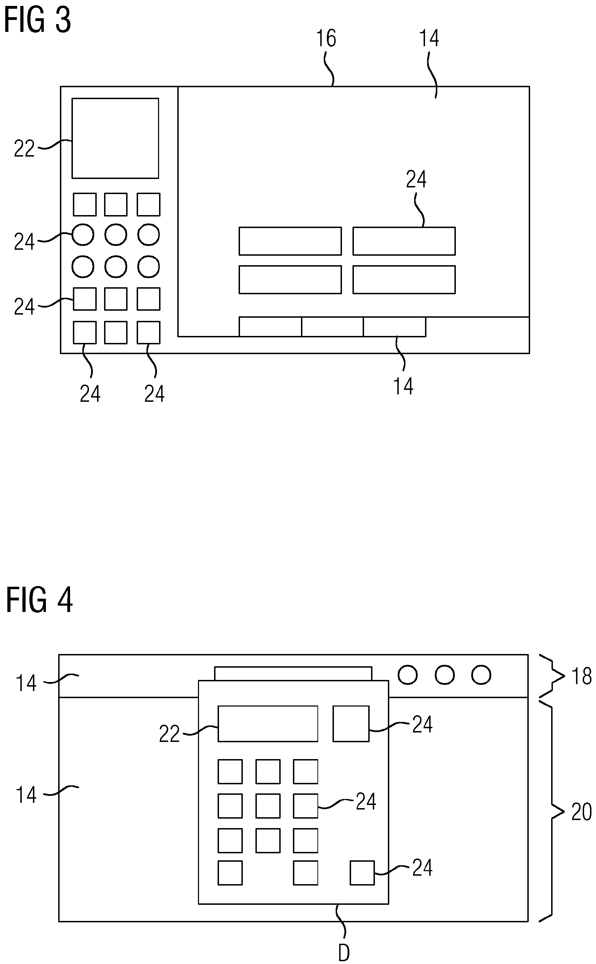

Eine beispielhafte Anzeige eines Clients 6 ist in FIG 2 gezeigt und ist hier eine hybriden Anzeige, bei welcher eine Anzeigefläche 16 des Clients 6 in zwei Anzeigebereiche 18, 20 unterteilt ist, nämlich einen ersten Bereich 18 für die Benutzeroberfläche 14 des Master-Geräts 2, in FIG 2 am oberen Rand der Anzeigefläche 16, und einen zweiten Bereich 20 für die Benutzeroberfläche 14 eines oder mehrerer Server 12, in FIG 2 unterhalb des ersten Bereichs 18. Im zweiten Bereich 20 wird nun je nach Bedarf die Benutzeroberfläche 14 eines jeweiligen Servers 12 anzeigt, speziell genau dann, wenn eine Interaktion eines Anwenders mit dem Server 12 erfolgen soll. Der zweite Bereich 20 ist aber auch umschaltbar zur Anzeige einer erweiterten Benutzeroberfläche 14 des Master-Geräts 2. Eine Variante für die Anzeige z.B. auf einem anderen der Clients 6 ist in FIG 3 gezeigt. Hier ist der zweite Bereich 20 über ein Register zugänglich.

In FIG 4 ist ausgehend von FIG 2 gezeigt, dass der Client 6 mit hybrider Anzeige zusätzlich derart ausgebildet, dass Anfragen des Master-Geräts 2 an den Anwender in Form von Dialogfenstern D im zweiten Bereich 20 angezeigt werden und dann die dort ggf. angezeigte Fremdanzeige überlagern, zumindest temporär, d.h. bis die notwendige Eingabe erfolgt ist. Dadurch wird für die Interaktion mit dem Master-Gerät 2 eine vergrößerte Anzeigefläche bereitgestellt.

Zur Übertragung einer Benutzeroberfläche 14 von einem Server 12 über den Proxy 4 auf einen Client 6 wird ein spezielles Verfahren verwendet, welches ein spezielles Übertragungsprotokoll implementiert, um eine besonders sichere Übertragung der Benutzeroberfläche 14 in einem möglichst einfachen Standard-Netzwerk durch eine protokollseitig definierte Latenz-überwachung auf Bildspeicherebene zu realisieren.

Gemäß dem Verfahren wird eine Benutzeroberfläche 14 eines Servers 12 an die Clients 6 zunächst grundsätzlich derart übertragen, dass die Benutzeroberfläche 14 auf dem Client 6 angezeigt wird und der Server 12 vom Client 6 aus steuerbar ist. Dadurch ist es für einen Anwender möglich, mittels des Clients 6 den Server 12 zu steuern und umgekehrt Informationen des Servers 12 an den Client 6 zu übermitteln und über diesen auszugeben. Allgemein ist vom Client 6 aus eine Interaktion, also sowohl Anzeige als auch Eingabe, mit dem Server 12 möglich. Die Benutzeroberfläche 14 umfasst typischerweise wie aus den FIG 2 bis 4 ersichtlich Anzeigefelder 22 zur Darstellung von Informationen sowie Bedienfelder 24 zur Eingabe von Steuerbefehlen. Die konkrete Ausgestaltung der Benutzeroberfläche 14 ist vom Gerät 2, 12 abhängig.

Gemäß dem Verfahren wird zur Anzeige der Benutzeroberfläche 14 auf dem Client 6 ein Datenstrom von mehreren aufeinanderfolgenden Bildern 26 vom Server 12 an den Client 6 mittels einer paketbasierten Übertragung übermittelt. Dies erfolgt vorliegend in Form von Paketen, wobei vorliegend ohne Beschränkung der Allgemeinheit angenommen wird, dass jedes Paket genau ein Bild 26 enthält. Der Datenstrom besteht demnach aus mehreren aufeinanderfolgenden Bildern, welche mit einer Frequenz ausgesendet werden, welche einer Bildwiederholrate des Servers 12 entspricht. Zusätzlich zu einem Bild 26 enthält ein Paket noch andere Daten, vorliegend eine Bildnummer.

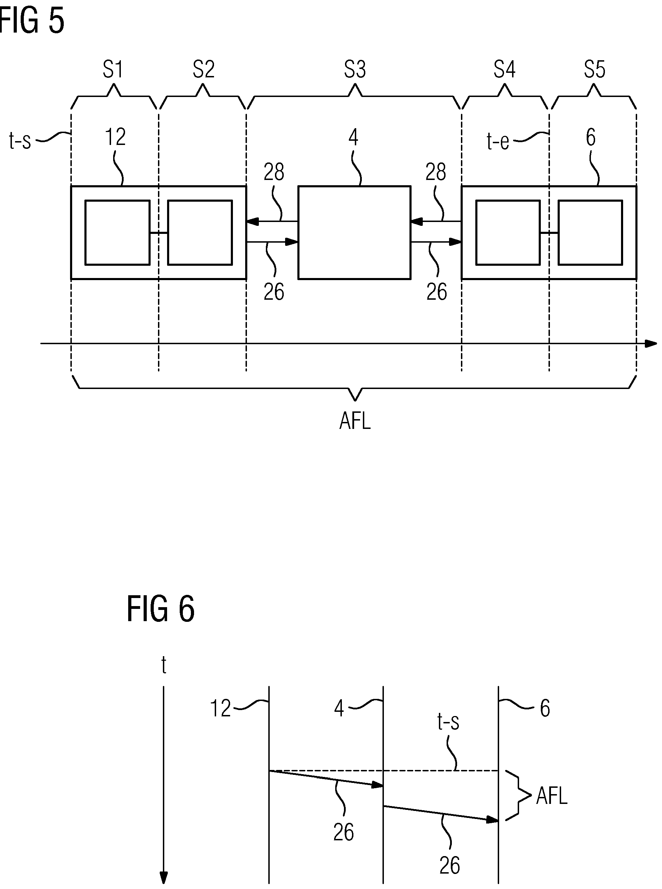

Die paketbasierte Übertragung ist vorliegend bidirektional. Dies ist in den FIG 5 bis 8 erkennbar. Ein Server 12 versendet ein Paket mit einem Bild 26 und anderen Daten über den Proxy 4 an die Clients 6, welche nach Empfang des Pakets eine Rückantwort 28, ebenfalls ein Paket, über den Proxy 4 an den Server 12 zurücksenden. Die Rückantwort 28 enthält die Bildnummer, sodass der Server 12 die Rückantwort 28 einem zuvor versendeten Paket zuordnen kann.

Gemäß dem Verfahren wird zur Überwachung der Übertragung eine Latenz AFL, IFL der Übertragung wiederkehrend bei Empfang einzelner Bilder 26 an einem jeweiligen Client 6 bestimmt. Durch diese Überwachung des Empfangs der einzelnen Bilder 26, ist eine Überwachung der Latenz AFL, IFL der Übertragung auf Bildspeicherebene realisiert. Dazu wird vorliegend der Empfang eines Bildes 26 durch einen Client 6 als Auslöser für eine Bestimmung der Latenz AFL, IFL verwendet, und zwar wiederkehrend, sodass die Latenz AFL, IFL fortlaufend bestimmt und dabei mit einer Frequenz gemessen wird, welche in der Größenordnung der Bildwiederholrate der Servers 12 und der Clients 6 liegt.

Die Bestimmung der Latenz AFL, IFL wird vorliegend dazu genutzt, eine Verlangsamung der Übertragung über die Netzwerke 8, 10 oder eine Überladung eines Clients 6 besonders schnell zu erkennen und eine entsprechende Reaktion des Servers 12, des Proxy 4 oder des Clients 6 hierauf auszulösen. Dabei wird eine Reaktion vorliegend bei Überschreiten eines vorgegebenen Schwellwerts für die Latenz AFL, IFL ausgelöst. Die Latenz AFL, IFL wird vom Client 6, vom Proxy 4 oder vom Server 12 oder von mehreren dieser Komponenten bestimmt. Die hierzu notwendigen Informationen werden dem Datenstrom, genauer gesagt den einzelnen Paketen entnommen. In der gezeigten Ausgestaltung mit mehreren Servern 12 und mehreren Clients 6 wird die Latenz AFL, IFL individuell für jede einzelne Verbindung zwischen einem der Server 12 und einem der Clients 6 und somit individuell für jeden Datenstrom erkannt.

Die Bestimmung der Latenz AFL, IFL ist protokollseitig definiert, also durch ein Übertragungsprotokoll, sodass keine speziellen Anforderungen an die Hardware vorliegen und das Verfahren mit besonders kostengünstiger und einfacher Standard-Technologie durchgeführt wird. Zumindest im externen Netzwerk 10 erfolgt die Übertragung der Benutzeroberfläche 14 drahtgebunden oder drahtlos über ein kostengünstiges, paketbasiertes Netzwerk, vorliegend z.B. Standard-Ethernet, WLAN oder dergleichen, und gerade nicht über ein spezielles Echtzeit-Ethernet. Das interne Netzwerk 8 basiert vorliegend z.B. auf TCP-Technologie. Das Übertragungsprotokoll dient auch zur Übermittlung von Steuerbefehlen an den jeweiligen Server 12 über einen entsprechenden Rückkanal.

Vorliegend wird die Latenz AFL, IFL vom Client 6 bestimmt und von diesem auch genutzt, um dessen Verhalten gegenüber dem Anwender geeignet anzupassen. Konkret wird der Anwender z.B. über Übertragungsprobleme informiert oder eine Eingabe über den Client 6 wird gesperrt, um Fehleingaben direkt zu verhindern. Auch laufende Eingaben werden unterbrochen, falls der Schwellwert überschritten wird. Der Schwellwert ist einstellbar und in einem Speicher des Clients 6 hinterlegt. Bei Überschreiten eines Schwellwerts wird zudem ein entsprechender Hinweis ausgegeben, beispielsweise ein Text, welcher darauf hinweist, dass die Latenz AFL, IFL den Schwellwert überschritten hat und die angezeigte Benutzeroberfläche 14 daher möglicherweise nicht mehr aktuell ist. Der Text wird beispielsweise als halbtransparente Überblendung der Benutzeroberfläche 14 angezeigt.

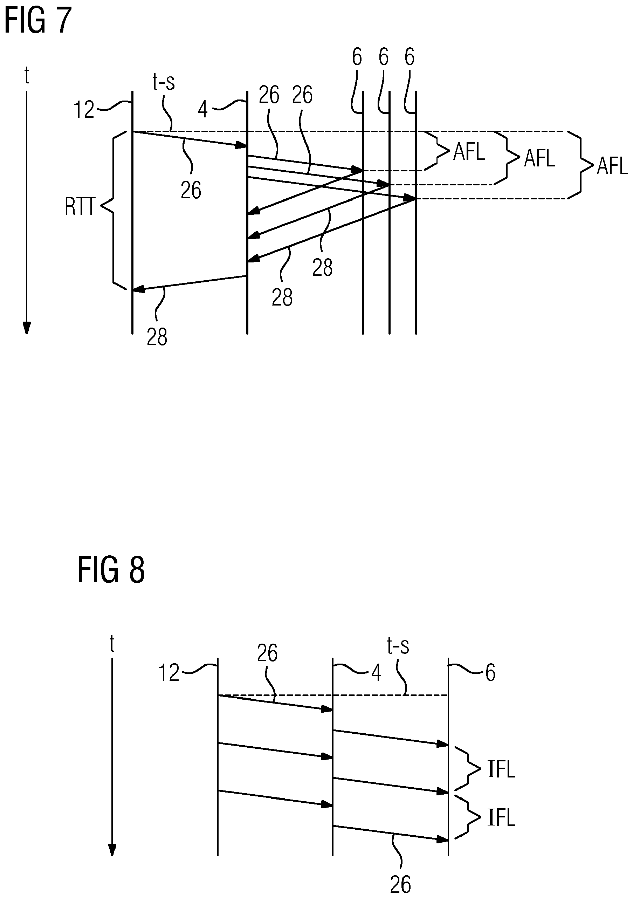

In den FIG 5 bis 8 sind diverse Konzepte zur Bestimmung der Latenz AFL, IFL gezeigt. Dabei ist in FIG 5 eine vereinfachte Darstellung der Architektur der FIG 1 mit einer Zeitachse für eine Zeit t gezeigt und in den FIG 6 bis 8 ist jeweils in vertikaler Richtung die Zeit t aufgetragen und in horizontaler Richtung die Verbindung von einem Server 12 über einen Proxy 4 zu einem oder mehreren Clients 6. In den FIG 5 bis 7 wird eine absolute Latenz AFL bestimmt. In FIG 8 wird clientseitig eine relative Latenz IFL bestimmt.

Der Server 12 sendet beim Übertragen eines jeweiligen Bildes 26 einen Zeitstempel mit, welcher eine Startzeit t-s der serverseitigen Verarbeitung des Bildes 26 angibt, nämlich den Beginn eines Einlesens S1 des Bildes 26 aus einem Bildspeicher des Servers 12. Die clientseitige Verarbeitung des Bildes 26 ist in einen Empfangsvorgang S4 und einen Anzeigevorgang S5 für das Bild 26 unterteilt ist, wobei der Anzeigevorgang S5 erst dann beginnt, wenn der Empfangsvorgang S4 beendet ist. Die absolute Latenz AFL wird dann als Summe aus zwei Zeitabschnitten bestimmt, nämlich einem ersten Zeitabschnitt, welcher von der Startzeit t-s bis zum Ende des Empfangsvorgangs S4 reicht, wobei dieses Ende insbesondere durch eine Endzeit t-e markiert ist, welche entsprechend festgestellt wird. Ein zweiter Zeitabschnitt gibt eine Dauer des Anzeigevorgangs S5 an. Die Endzeit t-e wird nach einem Empfang des jeweiligen Bildes 26 durch den Client 6 selbst festgestellt.

Die absolute Latenz AFL ergibt sich somit wie in FIG 5 gezeigt aus den folgenden, nacheinander ausgeführten Schritten 51 bis S5: erstens, ein serverseitiger Einlesevorgang S1 für ein Bild 26, nämlich ein Einlesen (grabben) des Bildes 26 aus dem Bildspeicher des Servers 12; zweitens, eine Komprimierung S2 des Bildes 26, welche allerdings optional ist; drittens, eine paketbasierte Übertragung S3 des Bildes 26 vom Server 12 über den Proxy 4 zum Client 6; viertens, ein Empfang und eine Dekodierung S4 des Bildes 26 durch den Client 6; fünftens, eine Anzeige S5 des Bildes 26 auf dem Client 6. Der erste und zweite Schritt S1, S2 sind dabei Bestandteile der serverseitigen Verarbeitung des Bildes 26. Der dritte Schritt S3 bezeichnet die Übertragung zwischen Server 12 und Client 6 über den Proxy 4 und die diversen Netzwerke 8, 10. Der vierte und fünfte Schritt S4, S5 sind Bestandteile der clientseitigen Verarbeitung des Bildes 26, wobei der vierte Schritt S4 dem Empfangsvorgang des Clients 6 entspricht und der fünfte Schritte S5 dem Anzeigevorgang. Jeder dieser Schritte weist eine gewisse Dauer auf und die Summe dieser Dauern ergibt die absolute Latenz AFL.

Die Dauer vom Beginn des ersten Schritts S1 bis zum Ende des vierten Schritts S4 wird anhand der Startzeit t-s und der Endzeit t-e errechnet und somit vollständig softwareseitig. Die Bestimmung der Dauer des fünften Schritts S5, d.h. des Anzeigevorgangs, wird entweder als ein vorbestimmter Offset in einem Speicher hinterlegt oder vom Client 6 für ein jeweiliges Bild 26 gemessen oder beides und dann entsprechend aufaddiert. Der Offset wird z.B. im Vorfeld durch entsprechende Versuche und Messungen ermittelt und dann im laufenden Betrieb abgefragt. Zur Messung der Dauer des Anzeigevorgangs S5 im laufenden Betrieb wird der Client 6 mit einem speziellen Anzeigetreiber betrieben, welcher laufend bei einem jeweiligen Bild 26 dynamisch die Dauer des Anzeigevorgangs S5 misst.

In einer nicht gezeigten Weiterbildung wird zusätzlich zur absoluten Latenz AFL noch eine Interaktionslatenz bestimmt, welche der bisher beschriebenen absoluten Latenz AFL vorausgeht und diejenige Dauer beschreibt, die benötigt wird, damit eine Eingabe des Anwenders am Client 6 am Server 12 zu einer Veränderung der Benutzeroberfläche 14 führt.

Bei mehreren Clients 6 ergeben sich für die unterschiedlichen Datenströme unter Umständen auch unterschiedliche absolute Latenzen AFL. FIG 7 zeigt, wie der Proxy 4 die absoluten Latenzen AFL mehrerer Clients 6 bezüglich eines Servers 12 auswertet und mittels eines Maximalvergleichs die größte absolute Latenz AFL bestimmt, welche dann an den Server 12 übertragen wird, sodass dieser geeignet reagieren kann. Wie in FIG 7 gezeigt ist, wird die absolute Latenz AFL in der Rückantwort 28 an den Server 12 übermittelt, welcher daraus in Verbindung mit der Ankunftszeit der Rückantwort 28 am Server 12 eine Umlaufzeit RTT für die Übertragung zwischen Server 12 und Client 6 berechnet. Die Umlaufzeit RTT ergibt sich hier als Summe aus der größten absoluten Latenz AFL und derjenigen Zeit, welche benötigt wird, bis die Rückmeldung 28 des langsamsten Clients 6 den Server 12 erreicht.

Zur Bestimmung der Startzeit t-s und der Endzeit t-e weisen der Server 12 und der Client 6 jeweils eine nicht gezeigte Echtzeituhr auf. Beim Verbindungsaufbau initialisiert der Server 12 in nicht gezeigter Weise einen monoton aufwärts zählenden Zähler, welcher auch zur Erzeugung der Zeitstempel verwendet wird. Dadurch ist eine virtuelle Uhr realisiert, mit welcher sich der Proxy 4 beim Verbindungsaufbau und im weiteren Verlauf der Verbindung wiederkehrend synchronisiert. Daraus ermittelt der Proxy 4 eine relative Abweichung seiner Zeit zur Zeit des Servers 12. Entsprechendes gilt für den Client 6. Auf eine tatsächliche Synchronisierung der Echtzeituhren wird dabei verzichtet. Die jeweilige relative Abweichung wird über mehrere Messungen gemittelt.

Die relative Latenz IFL (inter frame latency) wird als Zeit t zwischen zwei aufeinanderfolgenden Bildern bestimmt, z.B. wie in FIG 8 gezeigt. Die relative Latenz IFL ergibt sich z.B. als Differenz der Empfangszeiten zweier aufeinanderfolgender Bilder 26 am Client 6. Der Client 6 erkennt dadurch, ob regelmäßig eine Aktualisierung der Anzeige erfolgt oder ob die Übertragung eingefroren oder unterbrochen ist. Dadurch ist eine Aktivitätsprüfung realisiert, mittels welcher ein Verbindungsverlust erkannt wird, falls die relative Latenz IFL einen entsprechenden Schwellwert von z.B. 1 s überschreitet.

Der Datenstrom, welcher von einem Server 12 erzeugt wird, ist für das interne Netzwerk 8 oder den Client 6 oder beide möglicherweise zu umfangreich. Um eine zu starke Belastung der Bandbreite des Netzwerks sowie eine Überladung eines im Vergleich zum Server 12 leistungsschwachen Clients 6 zu vermeiden, wird eine Bandbreitensteuerung vorgenommen, bei welcher die Menge der Bilder 26 und somit die Größe des Datenstroms zwischen Server 12 und Client 6 insbesondere durch den Proxy 4 gesteuert werden. Durch diese Reduzierung der Bilddaten wird auch eine Störung der Übertragung anderer Daten vermieden, sodass eine zusätzliche Qualitätssicherung durch die Netzwerke 8, 10 nicht benötigt wird.

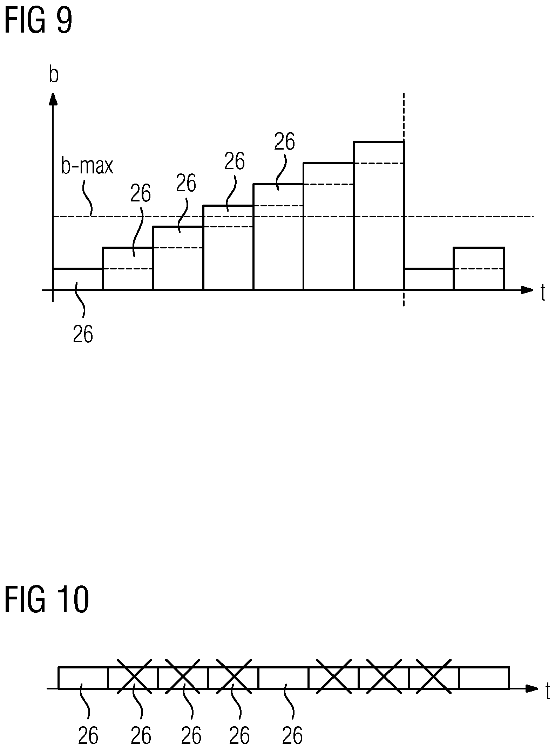

In den FIG 9 und 10 sind zwei Konzepte zur Begrenzung der Bandbreite beschrieben, nämlich in FIG 9 eine statische und in FIG 10 eine dynamische Bandbreitensteuerung. Gezeigt sind jeweils die gesendeten Bilder 26 als Funktion der Zeit t. Vorliegend sind die beiden Konzepte derart kombiniert, dass die dynamische Begrenzung verwendet wird, wenn zur statischen Begrenzung keine bestimmte Bildwiederholrate für den betreffenden Client 6 im Proxy 4 gespeichert ist.

FIG 9 zeigt die für nachfolgende Bilder 26 innerhalb eines Zeitabschnitts t1 aufsummierte Bandbreite b als Funktion der Zeit t. Allgemein versendet der Server 12 die Bilder 26 mit einer Bildwiederholrate, welche vom Proxy 4 gemäß FIG 9 auf eine maximale Bandbreite b-max des internen Netzwerks 8 begrenzt wird, indem der Proxy 4 innerhalb eines gegebenen Zeitabschnitts t1 lediglich solange Bilder 26 an den Client 6 weiterleitet, bis die maximale Bandbreite b-max erreicht ist. Alle nachfolgenden Bilder 26 innerhalb des Zeitabschnitts t1 von z.B. 1 s werden nicht an den Client 6 weiterleitet. Somit erfolgt sozusagen eine rollierende Summenbildung der Bilddatenmenge, welche übertragen wird, und ein Auslassen von Bildern 26 bei Überschreiten einer Obergrenze für diese Datenmenge. Die innerhalb des Zeitabschnitts t1 akkumulierte Datenmenge ist in FIG 9 durch die größer werdenden Balken illustriert. Die verworfenen Bilder 26 werden vom Proxy 4 nicht an den Client 6 weitergeleitet, in der Rückantwort 28 an den Server 12 wird jedoch vermerkt, ob das zugehörige Bild 26 verworfen wurde, sodass der Server 12 je nach Ausgestaltung entsprechend reagieren kann.

Bei der dynamischen Begrenzung gemäß FIG 10 leitet der Proxy 4 die Bilder 26 vom Server 12 mit einer Bildwiederholrate weiter, welche dynamisch an die Rechenzeit des Clients 6 angepasst wird, indem die Umlaufzeit RTT zwischen dem Server 12 und dem Client 6 ermittelt wird und abhängig von der Umlaufzeit RTT lediglich eine Teilmenge an Bildern 26 an den Client 6 weitergeleitet wird. In FIG 10 ist dies illustriert durch die zeitlich aufeinanderfolgenden Bilder 26, von welchen lediglich jedes vierte Bild 26 an den Client 4 weitergeleitet wird und die drei dazwischenliegenden Bilder 26 verworfen werden. Die Teilmenge an Bildern 26, welche weitergeleitet wird, ist durch einen einstellbaren Teiler bestimmt, welcher eine Anzahl aufeinanderfolgender Bilder 26 angibt, von welchem eines weitergeleitet wird und alle übrigen verworfen werden. In FIG 10 beträgt der Teiler demnach 4, sodass nur jedes vierte Bild 26 weitergeleitet wird. Die Bildwiederholrate wird nun abhängig von der Umlaufzeit RTT angepasst, indem der Teiler um 1 erhöht wird, falls die Umlaufzeit RTT einen oberen Schwellwert überschreitet, und um 1 verringert wird, falls die Umlaufzeit RTT einen unteren Schwellwert unterschreitet.

Solche Bilder 26, welche vom Client 6 zu einem gegebenen Zeitpunkt nicht angezeigt werden, werden zudem vorliegend vom Server 12 gar nicht erst übermittelt. Dies erfolgt im Rahmen einer weiteren Bandbreitensteuerung, bei welcher nicht benötigte Datenströme zu einem jeweiligen Client 6 deaktiviert werden, sodass weiter Bandbreite eingespart wird und das interne Netzwerk 8 und der jeweilige Client 6 entlastet werden. Alternativ oder zusätzlich transkodiert der Proxy 4 die Bilder 26, welche vom Server 12 zum Client 6 weitergeleitet werden, derart, dass deren Größe reduziert wird, um das interne Netzwerk 8 weiter zu entlasten.

## Patentansprüche

1. Verfahren zur Übertragung einer Benutzeroberfläche (14) von wenigstens einem Server (12) über einen Proxy (4) auf wenigstens einen Client (6),
wobei eine Benutzeroberfläche (14) des Servers (12) an den Client (6) derart übertragen wird, dass die Benutzeroberfläche (14) auf dem Client (6) angezeigt wird und der Server (12) vom Client (6) aus steuerbar ist,
wobei zur Anzeige der Benutzeroberfläche (14) auf dem Client (6) ein Datenstrom von mehreren aufeinanderfolgenden Bildern (26) vom Server (12) an den Client (6) mittels einer paketbasierten Übertragung übermittelt wird,
wobei zur Überwachung der Übertragung eine Latenz (AFL, IFL) der Übertragung wiederkehrend für einzelne Bilder (26) bestimmt wird,
wobei das Verfahren wenigstens einen der folgenden Schritte umfasst:
(i) wobei die Latenz (AFL) als absolute Latenz bestimmt wird, nämlich als Differenz zwischen einem Beginn einer serverseitigen Verarbeitung eines jeweiligen Bildes (26) und dem Ende einer clientseitigen Verarbeitung für das jeweilige Bild (26), die Benutzeroberfläche (14) des Servers (12) über den Proxy (4) auf mehrere Clients (6) übertragen wird, sodass für jeden Client (6) ein eigener Datenstrom vorliegt, jeder Client (6) für den jeweiligen Datenstrom die absolute Latenz (AFL) bestimmt und der Proxy (4) die größte absolute Latenz (AFL) bestimmt und an den Server (12) übermittelt,
(ii) wobei der Proxy (4) die Bilder (26) vom Server (12) mit einer Bildwiederholrate weiterleitet, welche dynamisch angepasst wird, indem eine Umlaufzeit (RTT) zwischen dem Server (12) und dem Client (6) ermittelt wird und abhängig von der Umlaufzeit (RTT) lediglich eine Teilmenge an Bildern (26) an den Client (6) weitergeleitet wird, die Teilmenge an Bildern (26), welche weitergeleitet wird, durch einen einstellbaren Teiler bestimmt ist, welcher eine Anzahl aufeinanderfolgender Bilder (26) angibt, von welchen eines weitergeleitet wird und alle übrigen verworfen werden, und der Teiler um 1 erhöht wird, falls die Umlaufzeit (RTT) einen oberen Schwellwert überschreitet, und um 1 verringert wird, falls die Umlaufzeit (RTT) einen unteren Schwellwert unterschreitet.

2. Verfahren nach Anspruch 1,
wobei der Server (12) beim Übertragen eines jeweiligen Bildes (26) einen Zeitstempel mitsendet, welcher eine Startzeit (t-s) der serverseitigen Verarbeitung des Bildes (26) angibt, nämlich den Beginn eines Einlesens des Bildes (26) aus einem Bildspeicher des Servers (12), wobei die clientseitige Verarbeitung in einen Empfangsvorgang und einen Anzeigevorgang für das Bild (26) unterteilt ist,
wobei die absolute Latenz (AFL) als Summe aus zwei Zeitabschnitten bestimmt wird, nämlich einem ersten Zeitabschnitt, welcher von der Startzeit (t-s) bis zum Ende des Empfangsvorgangs reicht, und einem zweiten Zeitabschnitt, welcher eine Dauer des Anzeigevorgangs angibt.

3. Verfahren nach Anspruch 2,
wobei die Dauer des Anzeigevorgangs als ein vorbestimmter Offset in einem Speicher hinterlegt ist,
oder
wobei die Dauer des Anzeigevorgangs vom Client (6) für ein jeweiliges Bild (26) gemessen wird.

4. Verfahren nach einem der Ansprüche 1 bis 3,
wobei der Server (12) die Bilder (26) mit einer Bildwiederholrate versendet, welche vom Proxy (4) auf eine maximale Bandbreite (b-max) begrenzt wird, indem der Proxy (4) innerhalb eines gegebenen Zeitabschnitts (t1) lediglich solange Bilder (26) an den Client (6) weiterleitet, bis die maximale Bandbreite (b-max) erreicht ist und alle nachfolgenden Bilder (26) innerhalb des Zeitabschnitts (t1) nicht an den Client (6) weiterleitet.

5. Verfahren nach einem der Ansprüche 1 bis 4,
wobei auf dem Client (6) zu einem gegebenen Zeitpunkt lediglich eine Teilanzahl von mehreren Datenströmen mehrerer Server (12) angezeigt wird und die übrigen Datenströme pausiert werden, sodass deren Bilder (26) nicht zum Client übertragen werden.

6. Verfahren nach einem der Ansprüche 1 bis 5,
wobei der Proxy (4) über wenigstens ein externes Netzwerk (10) mit dem Server (12) oder mit mehreren Servern (12) verbunden ist, wobei das Netzwerk (10) ein Standard-Ethernet-Netzwerk oder WLAN-Netzwerk ist.

7. Verfahren nach einem der Ansprüche 1 bis 6,
wobei der Server (12) ein erstes Medizingerät ist und
wobei der Proxy (4) und der Client (6) jeweils Teil eines zweiten Medizingeräts (2) sind und wobei der Client (6) ein Monitor ist, sodass eine Benutzeroberfläche (14) des ersten Medizingeräts auf den Monitor des zweiten Medizingeräts derart durchgeschleift wird, dass das erste Medizingerät vom zweiten Medizingerät (2) aus steuerbar ist.

8. Medizingerät (2), welches einen Proxy (4) und einen Client (6) aufweist und welches ausgebildet ist, eine Benutzeroberfläche (14) eines Servers (12) an den Client (6) derart zu übertragen, dass die Benutzeroberfläche (14) auf dem Client (6) angezeigt wird und der Server (12) vom Client (6) aus steuerbar ist, wobei zur Anzeige der Benutzeroberfläche (14) auf dem Client (6) ein Datenstrom von mehreren aufeinanderfolgenden Bildern (26) vom Server (12) an den Client (6) mittels einer paketbasierten Übertragung übermittelt wird, wobei zur Überwachung der Übertragung eine Latenz (AFL, IFL) der Übertragung wiederkehrend für einzelne Bilder (26) von dem Proxy (4) und dem Client (6) bestimmt wird, wobei das Medizingerät (2) ausgebildet ist, das Verfahren nach Anspruch 1 durchzuführen.

9. Anordnung, welche einen Server (12) und das Medizingerät (2) gemäß Anspruch 8 umfasst und zur Durchführung des Verfahrens nach einem der Ansprüche 1 bis 7 eingerichtet ist.

## Claims

1. Method for transferring a user interface (14) from at least one server (12) via a proxy (4) to at least one client (6),
wherein a user interface (14) of the server (12) is transferred to the client (6) such that the user interface (14) is displayed on the client (6), and the server (12) can be controlled from the client (6), wherein in order to display the user interface (14) on the client (6), a data stream of a plurality of successive frames (26) is transmitted from the server (12) to the client (6) by means of a packet-based transfer,
wherein a latency (AFL, IFL) of the transfer is determined recurrently for individual frames (26) in order to monitor the transfer,
wherein the method comprises at least one of the following steps:
(i) wherein the latency (AFL) is determined as the absolute frame latency, namely as the difference between a start of a server-side processing of a particular frame (26), and the end of a client-side processing of that particular frame (26), the user interface (14) of the server (12) is transferred via the proxy (4) to a plurality of clients (6), resulting in a dedicated data stream for each client (6), each client (6) determines the absolute frame latency (AFL) for the associated data stream, and the proxy (4) determines the largest absolute frame latency (AFL), which it transmits to the server (12),
(ii) wherein the frames (26) from the server (12) are forwarded by the proxy (4) at a frame rate that is adapted dynamically by ascertaining a round-trip time (RTT) between the server (12) and the client (6), and depending on the round-trip time (RTT), forwarding only a subset of frames (26) to the client (6), the subset of frames (26) that is forwarded is defined by an adjustable factor, which specifies a number of successive frames (26), of which one is forwarded and all the others are discarded, the factor is increased by 1 if the round-trip time (RTT) exceeds an upper threshold value, and decreased by 1 if the round-trip time (RTT) goes below a lower threshold value.

2. Method according to claim 1,
wherein when transferring a particular frame (26), the server (12) sends as well a time stamp that indicates a start time (t-s) of the server-side processing of the frame (26), namely the start of reading the frame (26) from a frame buffer of the server (12),
wherein the client-side processing is divided into a receive process and a display process for the frame (26), wherein the absolute frame latency (AFL) is determined as the sum of two time segments, namely a first time segment, which extends from the start time (t-s) until the end of the receive process, and a second time segment, which defines a time length of the display process.

3. Method according to claim 2,
wherein the time length of the display process is stored as a predefined offset in a memory,
or
wherein the client (6) measures the time length of the display process for a particular frame (26).

4. Method according to one of claims 1 to 3,
wherein the server (12) sends the frames (26) at a frame rate that is limited by the proxy (4) to a maximum bandwidth (b-max), by means of the proxy (4) forwarding frames (26) to the client (6) within a given time segment (t1) only until the maximum bandwidth (b-max) is reached, and not forwarding to the client (6) any of the subsequent frames (26) within the time segment (t1).

5. Method according to one of claims 1 to 4,
wherein at a given point in time only some of a plurality of data streams from a plurality of servers (12) are displayed on the client (6), and the remaining data streams are paused, so that their frames (26) are not transferred to the client.

6. Method according to one of claims 1 to 5,
wherein the proxy (4) is connected via an external network (10) to the server (12) or to a plurality of servers (12), wherein the network (10) is a standard Ethernet network or a WLAN network.

7. Method according to one of claims 1 to 6,
wherein the server (12) is a first medical device, and
wherein the proxy (4) and the client (6) are both part of a second medical device (2), and wherein the client (6) is a monitor, so that a user interface (14) of the first medical device is looped through to the monitor of the second medical device such that the first medical device can be controlled from the second medical device (2).

8. Medical device (2), which comprises a proxy (4) and a client (6), and which is designed to transfer a user interface (14) of a server (12) to the client (6) such that the user interface (14) is displayed on the client (6), and the server (12) can be controlled from the client (6), wherein in order to display the user interface (14) on the client (6), a data stream of a plurality of successive frames (26) is transmitted from the server (12) to the client (6) by means of a packet-based transfer, wherein a latency (AFL, IFL) of the transfer is determined recurrently for individual frames (26) by the proxy (4) and the client (6) in order to monitor the transfer, wherein the medical device (2) is designed to implement the method according to claim 1.

9. Arrangement, which comprises a server (12) and the medical device (2) according to claim 8 and which is configured to implement the method according to one of claims 1 to 7.

## Revendications

1. Procédé de transmission d'une interface (14) d'utilisateur d'au moins un serveur (12) à au moins un client (6) par l'intermédiaire d'un proxy (4),
dans lequel on transmet une interface (14) d'utilisateur du serveur (12) au client (6), de manière à afficher l'interface (14) d'utilisateur sur le client (6) et à pouvoir commander le serveur (12) par le client (6),
dans lequel pour l'affichage de l'interface (14) d'utilisateur sur le client (6), on transmet un flux de données de plusieurs images (26) successives du serveur (12) au client (6) au moyen d'une transmission à base de paquets,
dans lequel pour le contrôle de la transmission, on détermine une latence (AFL, IFL) de la transmission, de manière récurrente pour diverses images (26),
dans lequel le procédé comprend au moins l'un des stades suivants :
(i) dans lequel on détermine la latence (AFL) en tant que latence absolue, à savoir en tant qu'une différence entre un début d'un traitement du côté du serveur d'une image (26) respective et la fin d'un traitement du côté du client pour l'image (26) respective, on transmet l'interface (14) d'utilisateur du serveur (12) à plusieurs clients (6) par l'intermédiaire du proxy (4), de manière à avoir pour chaque client (6) son propre flux de données, chaque client (6) détermine pour le flux de données respectif la latence (AFL) absolue et le proxy (4) détermine la latence (AFL) absolue la plus grande et la transmet au serveur (12),
(ii) dans lequel le proxy (4) achemine les images (26) du serveur (12) à un taux de répétition d'image, qui est adapté dynamiquement, en déterminant un temps (RTT) de circulation entre le serveur (12) et le client (6) et en acheminant, en fonction du temps (RTT) de circulation, seulement une quantité partielle d'images (26) au client (6), la quantité partielle d'images (26), que l'on achemine, étant déterminée par un diviseur réglable, qui indique un nombre d'images (26) successives, dont l'une est acheminée et toutes les autres rejetées, et le diviseur est augmenté de 1 si le temps (RTT) de circulation dépasse une valeur de seuil supérieure et est diminué de 1 si le temps (RTT) de circulation devient inférieur à une valeur de seuil inférieure.

2. Procédé suivant la revendication 1,
dans lequel le serveur (12) envoie à la transmission d'une image (26) respective une estampille temporelle, qui indique un temps (t-s) de début du traitement de l'image (26) du côté du serveur, à savoir le début d'une lecture de l'image (26) dans une mémoire d'images du serveur (12), dans lequel le traitement du côté du client est subdivisé en une opération de réception et en une opération d'affichage de l'image (26),
dans lequel on détermine la latence (AFL) absolue comme somme de deux laps de temps, à savoir un premier laps de temps, qui va du temps (t-s) de début jusqu'à la fin de l'opération de réception et un deuxième laps de temps, qui indique une durée de l'opération d'affichage.

3. Procédé suivant la revendication 2,
dans lequel on met dans une mémoire la durée de l'opération d'affichage sous la forme d'un décalage défini à l'avance
où,
dans lequel la durée de l'opération d'affichage est mesurée par le client (6) pour une image (26) respective.

4. Procédé suivant l'une des revendications 1 à 3,
dans lequel le serveur (12) achemine les images (26) à un taux de répétition d'image, qui est limité par le proxy (4) à une largeur de bande (b-max) maximum par le fait que le proxy (4) achemine dans un laps de temps (t1) donné des images (26) au client (6), seulement jusqu'à ce que la largeur de bande (b-max) maximum est atteinte et n'achemine pas au client (6) des images (26) suivantes dans le laps de temps (t1).

5. Procédé suivant l'une des revendications 1 à 4,
dans lequel on indique sur le client (6) à un instant donné seulement un nombre partiel de plusieurs flux de données de plusieurs serveurs (12), et on met en pause les autres flux de données, de manière à ne pas transmettre leurs images (26) au client.

6. Procédé suivant l'une des revendications 1 à 5,
dans lequel le proxy (4) est relié au serveur (12) ou à plusieurs serveurs (12) par l'intermédiaire d'au moins un réseau (10) extérieur, dans lequel le réseau (10) est un réseau Ethernet standard ou un réseau WLAN.

7. Procédé suivant l'une des revendications 1 à 6,
dans lequel le serveur (12) est un premier appareil médical et dans lequel le proxy (4) et le client (6) sont respectivement un deuxième appareil (2) médical et dans lequel le client (6) est un moniteur de sorte qu'une interface (14) d'utilisateur du premier appareil médical est balayée sur le deuxième moniteur du deuxième appareil médical, de manière à pouvoir commander le premier appareil médical par le deuxième appareil (2) médical.

8. Appareil (2) médical, qui a un proxy (4) et un client (6) et qui est constitué pour transmettre une interface (14) d'utilisateur d'un serveur (12) au client (6), de manière à ce que l'interface (14) d'utilisateur soit affichée sur le client (6) et de manière à ce que le serveur (12) puisse être commandé par le client (6), dans lequel, pour l'affichage de l'interface (14) d'utilisateur sur le client (6), un flux de données de plusieurs images (26) successives est transmis du serveur (12) au client (6), au moyen d'une transmission à base de paquets, dans lequel, pour le contrôle de la transmission, on détermine une latence (AFL, IFL) de la transmission de manière récurrente pour diverses images (26) par le proxy (4) et le client (6), l'appareil (2) médical étant constitué pour effectuer le procédé suivant la revendication 1.

9. Agencement, qui comprend un serveur (12) et l'appareil (2) médical suivant la revendication 8 et qui est agencé pour effectuer le procédé suivant l'une des revendications 1 à 7.
